(19) Europäisches Patentamt
European Patent Office
Office européen des brevets

(11) **EP 2 735 323 B1**

(12) **EUROPÄISCHE PATENTSCHRIFT**

(45) Veröffentlichungstag und Bekanntmachung des Hinweises auf die Patenterteilung:
**29.07.2015 Patentblatt 2015/31**

(51) Int Cl.:
*A61M 1/16* *(2006.01)*     *A61M 1/34* *(2006.01)*
*B01D 61/22* *(2006.01)*     *B01D 65/10* *(2006.01)*

(21) Anmeldenummer: **13194271.6**

(22) Anmeldetag: **25.11.2013**

(54) **Verfahren und Vorrichtung zur Erkennung einer verminderten Dialyseleistung verursacht durch Verklottung**

Method and apparatus for detecting decreased dialysis performance due to clotting

Procédé et dispositif de détection d'une puissance de dialyse réduite

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.11.2012 DE 102012111375**

(43) Veröffentlichungstag der Anmeldung:
**28.05.2014 Patentblatt 2014/22**

(73) Patentinhaber: **B. Braun Avitum AG**
**34212 Melsungen (DE)**

(72) Erfinder: **Ahrens, Jörn**
**34225 Baunatal (DE)**

(74) Vertreter: **Winter, Brandl, Fürniss, Hübner, Röss, Kaiser, Polte - Partnerschaft mbB**
**Patent- und Rechtsanwaltskanzlei**
**Bavariaring 10**
**80336 München (DE)**

(56) Entgegenhaltungen:
**EP-A1- 1 364 666     WO-A1-2004/002553**
**WO-A1-2004/073772     US-A- 6 039 877**

**Beschreibung**

Die vorliegende Erfindung beschreibt eine Vorrichtung und ein Verfahren zur Detektion von Verklottung eines Dialysators in einer Vorrichtung zur extrakorporalen Reinigung von Blut, indem blutseitig mindestens ein Drucksensor angeordnet ist, um den Druck im Blutkreislauf zu bestimmen und eine UV-Messeinrichtung im Dialysatausfluss die Absorbanz mindestens einer harnpflichtigen Substanz bestimmt, und aus gemessenen Druckmesswerten eine als Basislinie bezeichnete Ausgleichsgerade berechnet wird und ein Toleranzbereich für die gemessenen Druckmesswerte bestimmt wird und aus den gemessenen UV-Intensitäten zukünftige Werte berechnet werden und bestimmt wird, ob die gemessenen UV-Intensitäten und Druckmesswerte von den berechneten UV-Intensitäten und dem Toleranzbereich für die gemessenen Druckmesswerte um die Basislinie abweichen.

Bei Patienten mit eingeschränkter oder fehlender Nierenfunktion werden Abfallprodukte des natürlichen Stoffwechsels einschließlich urämischer Toxine durch Dialyseverfahren entfernt, wobei die Entfernung der Stoffe aus dem Blut, welches extrakorporal geführt wird, durch den Kontakt des Blutes mit einer Dialysierflüssigkeit erfolgt.

Die Reinigung des Blutes erfolgt durch diffusiven und konvektiven Stofftransport zwischen Dialysat und Blutseite. Auf Grund des Konzentrationsgradienten zwischen Blut und Dialysierflüssigkeit, die vorzugsweise im Gegenstromprinzip den Filter durchströmen, erfolgt der diffusive Konzentrationsausgleich und somit Entzug von Giftstoffen. Parallel wird durch Unterdruck auf der Dialysierflüssigkeitsseite dem Patienten Flüssigkeit beim konvektiven Stofftransport entzogen.

Ein entscheidender Prozess ist die Interaktion der Filtermembran mit Blut. Durch diese Wechselwirkung verschlechtern sich die Flusseigenschaften des Filters sowohl in Transmembranrichtung als auch in Blutflussrichtung. Diese Veränderungen werden beispielsweise durch Thrombozytenanlagerung auf die Membran, Koagelbildung, eine zu geringe Zugabe von Gerinnungshemmern, chemisches Binden von Blutbestandteilen an die Membran oder schlicht mechanisches (strömungsbedingtes) Drücken der Blutbestandteile an und sogar in die Poren verursacht.

Bei der Koagelbildung entsteht ein gallertartiges durch Fibrinfäden stabilisiertes Aggregat aus roten Blutkörperchen (Erythrozyten). Anders als der Begriff Thrombus beschreibt ein Koagulum ein Blutgerinnsel, welches sich außerhalb eines Blut- oder Lymphgefäßes (extravasal) befindet und nicht innerhalb (intravasal).

Besonders die Behandlung durch Hämodiafiltration wird dadurch beeinträchtigt, da hier auf den konvektiven Stofftransport mittelmolekularer Substanzen gesetzt wird. Durch Verschlechterung der transmembranen Flusseigenschaften oder der Permeabilität, verschlechtert sich auch der Siebkoeffizient für die urämischen Substanzen im mittelmolekularen Bereich, was dazu führt, dass bei gleicher Menge konvektiv gefilterter Flüssigkeit weniger urämische Substanzen aus dem Blutkreislauf entfernt werden. Ein anderer Effekt ist die Verringerung der effektiven Durchflussfläche sowohl in Blutflussrichtung als auch in Transmembranrichtung verursacht durch die Verschließung von Kapillaren, so dass die effektive Oberfläche zur Reinigung geringer ist. Dies hat einen direkten Effekt auf die Clearance des Dialysators und somit den diffusiven Stofftransport, welche direkt proportional zur Effektivität der Dialyse ist.

Um sicherzustellen, dass sich die Dialyseleistung während der gesamten Therapie nicht verringert und somit eine effektive Dialyse gewährleistet werden kann, ist es vorteilhaft, eine Verklottung des Dialysators möglichst frühzeitig zu erkennen.

In der Praxis wird eine Verklottung zumeist dadurch erkannt, dass eine Leitfähigkeitsmessung vor und hinter dem Dialysator vorgenommen wird, um die Natriumclearance des Dialysators zu bestimmen. Die Messung erfolgt dabei durch eine Unterbrechung der Behandlung in längeren Intervallen und durch eine punktuelle Messung der Natriumclearance. Die Natriumclearance ist der Harnstoffclearance sehr ähnlich und wird daher als Referenz für die Reinigungsfähigkeit des Dialysators genutzt.

Durch dieses Verfahren kann die Dialyseeffektivität durch die Näherung mit Hilfe der Natriumclearance punktuell festgestellt werden, wobei aber folgende Beschränkungen vorhanden sind:

- Störungen zwischen den Messintervallen bleiben zunächst verborgen bzw. können nicht identifiziert werden;
- Die Natriumclearance ist nur eine Näherung zur Harnstoffclearance,
- Jede Messung führt automatisch zu einer Beeinflussung der Behandlung.

Ein alternatives Verfahren bzw. eine alternative Vorrichtung zur Detektion einer Verklottung eines Dialysators in einer Vorrichtung zur extrakorporalen Reinigung von Blut wird in dem Dokument WO2004/002553 (A1) beschrieben. Es offenbart ein Verfahren zur Detektion von Verklottung eines Dialysators, wobei blutseitig an mindestens zwei unabhängigen Drucksensoren der Druck gemessen wird und aus einer Anzahl gemessener Druckmesswerte ein Basiswert berechnet wird, und wobei Verklottung detektiert wird, wenn ein aus gemessenen Druckmesswerten und anderen Parametern berechneter "clogging factor" einen Grenzwert überschreitet.

J Aufgabe der Erfindung ist es daher, ein Verfahren bereitzustellen, welches es erlaubt, eine Verklottung eines Dialysators während einer laufenden Dialysebehandlung eindeutig zu erkennen, ohne die Dialysebehandlung zu beeinträchtigen und den Nachteilen des Stands der Technik abzuhelfen.

Es wurde überraschend gefunden, dass diese Aufgabe gelöst wird, indem ein Verfahren gemäß einem der Ansprüche 1 bis 3 durchgeführt wird und eine Vorrichtung gemäß einem der Ansprüche 7 bis 9 bereitgestellt wird. Weitere vorteilhafte Ausgestaltungen der Erfindung sind in den abhängigen Patentansprüchen, der Beschreibung, den Figuren sowie den Beispielen offenbart.

Die vorliegende Erfindung betrifft daher ein Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein Toleranzbereich um die Basislinie und ein Toleranzbereich für die gemessenen UV-Absorbanzen berechnet wird und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{(gemessen)}$) innerhalb des berechneten Toleranzbereichs um die Basislinie liegt und ob die gemessene UV-Absorbanz ($A_{y+1(gemessen)}$) innerhalb des berechneten Toleranzbereichs für die berechnete UV-Absorbanz ($A_{y+1(berechnet)}$) liegt, wobei Verklottung detektiert wird, wenn der gemessene Druckmesswert ($P_{(gemessen)}$) außerhalb des berechneten Toleranzbereichs um die Basislinie liegt und die gemessene UV-Absorbanz ($A_{y+1(gemessen)}$) außerhalb des berechneten Toleranzbereichs für die berechnete UV-Absorbanz ($A_{y+1(berechnet)}$) liegt.

Gegenstand der Erfindung ist daher die Verknüpfung von blutseitigen Druckmessungen mit der Messung der UV-Absorbanz mindestens einer harnpflichtigen Substanz im Dialysatabfluss, um Verklottung zu detektieren. Dabei ist zu beachten, dass die UV-Absorbanz sich im Verlaufe der Dialysesitzung ändert, d.h. bei unveränderter Blutflussrate abnimmt.

Etwas anders formuliert betrifft die vorliegende Erfindung ein Verfahren zur Detektion von Verklottung des Dialysators in einer Vorrichtung zur extrakorporalen Reinigung von Blut, wobei blutseitig an mindestens einem Drucksensor der Druck gemessen BBM-P03492EP04 Anmeldung wird und im Dialysatabfluss die UV-Absorbanz mindestens einer harnpflichtigen Substanz gemessen wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein oberer Grenzwert über der Basislinie und ein unterer Grenzwert für die gemessenen UV-Absorbanzen berechnet wird und aus einer vordefinierten Anzahl (m) bestimmter UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{(gemessen)}$) den Grenzwert über der Basislinie übersteigt und ob die bestimmte UV-Absorbanz ($A_{y+1(gemessen)}$) den Grenzwert für die berechnete UV-Absorbanz ($A_{y+1(berechnet)}$) unterschreitet, wobei Verklottung detektiert wird, wenn der gemessene Druckmesswert ($P_{(gemessen)}$) den Grenzwert über der Basislinie übersteigt und die gemessene UV-Absorbanz ($A_{y+1(gemessen)}$) den Grenzwert für die berechnete UV-Absorbanz ($A_{y+1(berechnet)}$) unterschreitet.

Nimmt man idealer Weise an, dass sich der blutseitig gemessene Druck während der Dialysesitzung nicht ändert sofern die Blutflussrate unverändert ist und der Druck somit einer horizontalen Basislinie folgt, welche einen konstanten Druck bezeichnet, dann steigt der Druck bei einer auftretenden Verklottung an. Somit wird erfindungsgemäß angenommen, dass sich der blutseitige Druck während der Dialysesitzung nicht ändert und die UV-Absorbanz einer abfallenden Kurve folgt, so kann erfindungsgemäß Verklottung detektiert werden, wenn der Druck über ein gewisses Toleranzintervall oder über eine gewisse Obergrenze oberhalb der Basislinie ansteigt und die UV-Absorbanz entlang des abfallenden Kurvenverlaufs unterhalb eines gewissen Toleranzbereichs oder unterhalb einer gewissen unteren Grenze abfällt. Nimmt man nun an, dass der blutseitige Druck nur annähernd konstant bleibt sich aber auch im Laufe der Dialysesitzung leicht ändern kann, ohne dass Verklottung vorliegt, dann beschreibt die folgende Ausführungsform die Erfindung betreffend ein Verfahren zur Detektion von Verklottung des Dialysators in einer Vorrichtung zur extrakorporalen Reinigung von Blut, wobei blutseitig an mindestens einem Drucksensor der Druck bestimmt wird und im Dialysatabfluss die UV-Absorbanz mindestens einer harnpflichtigen Substanz gemessen wird und ein Toleranzbereich für die gemessenen Druckmesswerte und ein Toleranzbereich für die gemessenen UV-Absorbanzen berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{x+1(gemessen)}$) innerhalb des berechneten Toleranzbereichs für den berechneten Druckmesswert ($P_{x+1(berechnet)}$) liegt und ob die gemessene UV-Absorbanz ($A_{y+1(gemessen)}$) innerhalb des berechneten Toleranzbereichs für die berechnete UV-Absorbanz ($A_{y+1(berechnet)}$) liegt, wobei Verklottung detektiert wird, wenn der gemessene Druckmesswert ($P_{x+1(gemessen)}$) außerhalb des berechneten Toleranzbereichs für den berechneten Druckmesswert ($P_{x+1(berechneten)}$) liegt und die gemessene UV-Absorbanz ($A_{y+1(gemessen)}$) außerhalb des berechneten Toleranzbereichs für die berechnete UV-Absorbanz ($A_{y+1(berechnet)}$) liegt.

Ebenso umfasst die Erfindung ein Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und durch eine vordefinierte Anzahl (n) gemessener Druckmesswerte ($P_x$), worin x die Anzahl an Druckmessungen bezeichnet, eine als Basislinie bezeichnete Ausgleichsgerade gelegt wird, und ein Toleranzbereich um die Basislinie und ein Toleranzbereich für die gemessenen UV-Absorbanzen berechnet wird und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{(gemessen)}$) innerhalb des berechneten Toleranzbereichs um die Basislinie liegt und ob die gemessene UV-Absorbanz ($A_{y+1(gemessen)}$) innerhalb des berechneten Toleranzbereichs für die berechnete UV-Absorbanz ($A_{y+1(berechnet)}$) liegt, wobei Verklottung detektiert wird, wenn der gemessene Druckmesswert ($P_{(gemessen)}$) außerhalb des berechneten Toleranzbereichs um die Basislinie liegt und die gemessene UV-Absorbanz ($A_{y+1(gemessen)}$) außerhalb des berechneten Toleranzbereichs für die berechnete UV-Absorbanz ($A_{y+1(berechnet)}$) liegt.

Geht man näherungsweise davon aus, dass der blutseitige Druck im Verlaufe der Dialysesitzung konstant bleibt, so ist es nur einmal erforderlich, diesen Druck durch einige Druckmesswerte ($P_x$) zu bestimmen. n kann vorzugsweise zwischen 10 und 1.200 liegen. Innerhalb der ersten Minuten oder auch nur innerhalb der ersten Minute während der Dialysesitzung kann mit einer geringen Anzahl n gemessener Druckmesswerte ($P_x$) der Verlauf der Basislinie bestimmt werden. Der konstante Druckwert, hierin auch als Normwert bezeichnet, der Basislinie bleibt unverändert, solange die Blutflussrate unverändert bleibt und muss daher nur nach Änderung der Blutflussrate erneut bestimmt werden. Da angenommen wird, dass die Basislinie konstant bleibt, können selbst nach ein oder mehreren Stunden gemessene Druckmesswerte ($P_x$) mit dem Normwert verglichen werden, um Verklottung zu detektieren. Geht man jedoch davon aus, dass sich der blutseitig Druck während der Dialysesitzung ändern kann, z.B. durch Verdünnung des Blutes, so folgt der blutseitig Druck keiner konstanten Basislinie und zur Extrapolation des Druckverlaufs sind wiederkehrende oder kontinuierliche oder regelmäßige Druckmessungen einer Anzahl n an $P_x$-Druckmesswerten erforderlich. Dies berücksichtigt die folgende Ausführungsform der vorliegenden Erfindung betreffend ein Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und ein oberer Grenzwert für die gemessenen Druckmesswerte und ein unterer Grenzwert für die gemessenen UV-Absorbanzen berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) bestimmter UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{x+1(gemessen)}$) den Grenzwert für den berechneten Druckmesswert ($P_{x+1(berechnet)}$) übersteigt und ob die bestimmte UV-Absorbanz ($A_{y+1(gemessen)}$) den Grenzwert für die berechnete UV-Absorbanz ($A_{y+1(berechnet)}$) unterschreitet, wobei Verklottung detektiert wird, wenn der gemessene Druckmesswert ($P_{x+1(gemessen)}$) den Grenzwert für den berechneten Druckmesswert ($P_{x+1(berechnet)}$) übersteigt und die bestimmte UV-Absorbanz ($A_{y+1(gemessen)}$) den Grenzwert für die berechnete UV-Absorbanz ($A_{y+1(berechnet)}$) unterschreitet.

Ebenso umfasst die Erfindung ein Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz durch Messung der Transmission der eingestrahlten UV-Strahlung bestimmt wird und ein oberer Grenzwert für die gemessenen Druckmesswerte und ein oberer Grenzwert für die gemessene Transmission berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) gemessener Transmissionen ($T_y$) durch Extrapolation eine zukünftige Transmission ($T_{y+1(berechnet)}$) berechnet wird, worin y die Anzahl an UV-Messungen bezeichnet und bestimmt wird, ob der gemessene Druckmesswert ($P_{x+1(gemessen)}$) den Grenzwert für den berechneten Druckmesswert ($P_{x+1(berechnet)}$) übersteigt und ob die gemessene Transmission ($T_{y+1(gemessen)}$) den Grenzwert für die berechnete Transmission ($T_{y+1(berechnet)}$) übersteigt, wobei Verklottung detektiert wird, wenn der gemessene Druckmesswert ($P_{x+1(gemessen)}$) den Grenzwert für den berechneten Druckmesswert ($P_{x+1(berechnet)}$) übersteigt und die gemessene Transmission ($T_{y+1(gemessen)}$) den Grenzwert für die berechnete Transmission ($T_{y+1(berechnet)}$) übersteigt.

Eine bevorzugte Ausführungsform umfasst ein Verfahren zur Detektion von Verklottung des Dialysators in einer Vorrichtung zur extrakorporalen Reinigung von Blut, wobei blutseitig an mindestens einem Drucksensor ein Druck gemessen wird und im Dialysatabfluss die UV-Absorbanz mindestens einer harnpflichtigen Substanz durch Messung der Transmission der eingestrahlten UV-Strahlung bestimmt wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein oberer Grenzwert über der Basislinie und ein oberer Grenzwert für die gemessene Transmission berechnet wird und aus einer vordefinierten Anzahl (m) gemessener Transmissionen ($T_y$) durch Extrapolation eine zukünftige Transmission ($T_{y+1(berechnet)}$) berechnet wird, worin y die Anzahl an UV-Messungen bezeichnet und bestimmt wird, ob der gemessene Druckmesswert ($P_{(gemessen)}$) den Grenzwert über der Basislinie übersteigt und ob die gemessene Transmission ($T_{y+1(gemessen)}$) den Grenzwert für die berechnete Transmission ($T_{y+1(berechnet)}$) übersteigt, wobei Verklottung detektiert wird, wenn der gemessene Druckmesswert ($P_{(gemessen)}$) den Grenzwert über der Basislinie übersteigt und die gemessene Transmission ($T_{y+1(gemessen)}$) den Grenzwert für die berechnete Transmission ($T_{y+1(berechnet)}$) übersteigt.

Die Verfahren beruhen darauf, dass zwei unterschiedliche Messmethoden angewandt werden, die unabhängig voneinander sind. Erfindungsgemäß werden diese beiden Messmethoden miteinander kombiniert, da jede Messmethode für sich alleine keine eindeutige Detektion der Verklottung erlaubt. Erst die erfindungsgemäße Kombination beider Messmethoden macht die eindeutige Detektion von Verklottung möglich.

Beiden Methoden ist es gemeinsam, dass man bei einer eigenständigen Durchführung keine klare Aussage geben kann, da wichtige Umgebungseinflüsse nicht berücksichtigt werden. Erst durch das System aus Druckmessung, Absorbanzmessung und Abgleich der Werte mit Toleranzwerten können weitere Einflussgrößen erkannt und berücksichtigt werden, wodurch es möglich ist, eine eindeutige Aussage zu erreichen.

Die Überwachung des Drucks im extrakorporalen Blutkreislauf, und besonders über den Filter gibt an, ob sich der Durchflusswiderstand ändert. Dies kann durch Verklottung von ganzen Kapillaren aber auch durch andere Effekte wie der Bildung einer massiven Sekundärmembran erfolgen. Somit zeigt ein Druckanstieg nicht zwingend eine reduzierte Filterleistung.

Bei der alleinigen Überwachung der Verklottung durch Überwachung der Reduktion der Stoffkonzentration "harnpflichtiger" Substanzen im Dialysatabfluss mittels UV-Absorbanzmessung geht man von einer konstanten Übertragungsfunktion aus, die durch eine konstante Clearance bei konstantem Blutfluss angenommen wird. Ein Rückschluss auf Änderungen im System kann daher nicht eine Änderung der Übertragungsfunktion ausschließen. Damit haben beide Methoden den Nachteil, dass man unter optimalen Umständen eine Verklottung erkennen, aber nicht eine Veränderung der anderen Systemparameter bzw. Störungen ausschließen kann.

Das erfindungsgemäße Verfahren, dass auf einer Kombination der Drucküberwachung und der Ermittlung der UV-Verlaufskurve beruht, ermöglicht eine optimierte Aussage über ein mögliches Vorliegen der Verklottung, und filtert dabei Störungen der Umgebung bzw. des Systems raus. Dies geht sonst nicht. Dabei wird nicht nur eine parallele Überwachung verschiedener Parameter durchgeführt, sondern das entscheidende ist die Korrelation der beiden Eingangsmuster, die zeitlich verschoben in Korrelation gesetzt werden müssen.

Parallel dazu ist dies keine rein mathematische Betrachtung, sondern soll schlussendlich dem Patienten eine optimale Reinigungsleistung ermöglichen. Sofern also der Patient einen deutlichen Druckanstieg hat, kann dies auch andere Ursachen statt einer Verklottung haben, die jedoch keinen Verlust an Reinigungsleistung mit sich bringen und daher keinen Abbruch der Therapie erfordern, solange die Drücke noch im akzeptablen Bereich sind. Ebenso kann durch eine Veränderung von patientenspezifischen Parametern, wie der Clearance der Kompartimente oder anderer Organe die effektive Reinigungsmenge schwanken, ohne dass die Funktion des Dialysators beeinträchtigt ist. Auf Grund des erfindungsgemäßen Verfahrens kann in einem solchen Fall ebenfalls die Behandlung fortgeführt werden, da das System selbst keine Defizite hat.

Der Begriff Verklottung, wie hierin verwendet, beschreibt den Umstand, dass sich im Dialysator Kapillaren zusetzen und damit die zur Blutreinigung zur Verfügung stehende Fläche verringert ist. Das Zusetzen der Kapillaren kann dabei vielfältige Ursachen haben und umfasst unter anderem Thrombozytenanlagerung an die Kapillaren, Koagelbildung, eine zu geringe Zugabe von Gerinnungshemmern, chemisches Binden von Blutbestandteilen an die Kapillare oder schlicht mechanisches (strömungsbedingtes) drücken der Blutbestandteile an und sogar in die Kapillaren.

Bei der ersten Messmethode handelt es sich um die Messung des Drucks an mindestens einem Drucksensor im Blutkreislauf der extrakorporalen Blutbehandlungseinheit. Die Messung erfolgt bevorzugt blutseitig vor dem Dialysator über einen arteriellen Zulaufdrucksensor (9). Die Druckmessung vor dem Dialysator zeigt dabei den durch den Blutfluss erzeugten Eingangsdruck vor dem Dialysator an. Kommt es nun zu einer Verklottung des Dialysators erhöht sich der vor dem Dialysator gemessene Druckwert. Weiterhin kann der Druckmesswert auch blutseitig hinter dem Dialysator zum Ausschluss weiterer Effekte gemessen werden. Die Messung erfolgt dann bevorzugt über einen venösen Drucksensor (11). Dabei ist es bevorzugt, wenn die Anzahl n gemessener Druckmesswerte ($P_x$) mindestens 2 beträgt.

Bevorzugt kann auch an mindestens zwei oder weiteren Drucksensoren der Druck im Blutkreislauf der extrakorporalen Blutbehandlungseinheit gemessen werden. Die Messung mittels zweier oder weiterer Drucksensoren umfasst dabei die Messung zweier bzw. weiterer Druckmesswerte an zwei bzw. weiteren unterschiedlichen Drucksensoren, d.h. jeweils mindestens einen Druckmesswert an je einem Drucksensor. Somit ist es innerhalb der erfindungsgemäßen Verfahren möglich, dass an einem Drucksensor zwei oder mehrere Druckmesswerte gemessen werden, sowie dass an unterschiedlichen Drucksensoren Druckmesswerte gemessen werden. Die Anzahl der einzelnen Druckmesswerte, die an einem Drucksensor gemessen werden, können sich dabei von Drucksensor zu Drucksensor unterscheiden, wobei es vorteilhaft ist, wenn bei der Messung mehrerer Druckmesswerte auch die gleiche Anzahl an Druckmesswerten pro Drucksensor gemessen werden.

Bei den gemessenen Druckmesswerten kann es sich um Absolutdrücke und/oder relative Drücke handeln.

Der Begriff "Absolutdruck" oder "Absolutdrücke", wie hierin verwendet, beschreibt den Druck gegenüber dem Atmosphärendruck.

Der Begriff "relativer Druck" oder "relative Drücke", wie hierin verwendet, beschreibt die relative Änderung eines Druckmesswerts in Bezug auf einen zweiten Druckmesswert.

Werden mehrere Druckmesswerte an unterschiedlichen Drucksensoren gemessen, dann können diese untereinander in Relation gesetzt werden. Es können z.B. die absoluten Druckdifferenzen zwischen zwei Druckmesswerten oder relative Druckdifferenzen zwischen zwei Druckmesswerten oder absolute Druckamplituden bzw. relative Druckamplituden gebildet werden. Vorteilhaft ist es auch, die Differenz zwischen den absoluten Druckamplituden zweier Druckmesswerte oder die Differenz zwischen den relativen Druckamplituden zweier Druckmesswerte oder eine Kombination der oben genannten oder die Frequenzspektra der Druckmesswerte zu bilden. Einer oder mehrere dieser Parameter werden dabei überwacht.

Der Begriff "Druckdifferenz" oder "Druckdifferenzen", wie hierin verwendet, beschreibt die Differenz zweier Druckmesswerte.

Der Begriff "Druckamplitude" oder "Druckamplituden", wie hierin verwendet, beschreibt den ermittelten oder gemessenen Wert der Druckschwankungen.

Der Begriff "Frequenzspektrum" oder "Frequenzspektra", wie hierin verwendet, bezeichnet die Gesamtheit der Frequenzen, die von einem schwingenden System erzeugt werden bzw. in einem Signal enthalten sind.

Bevorzugt wird die Differenz und/oder das Verhältnis und/oder die Änderung der Amplitude und/oder das Verhältnis der Amplituden der gemessenen Druckmesswerte des arteriellen Zulaufdrucksensors (9) und des venösen Drucksensors (11) gebildet vorzugsweise unter Berücksichtigung der zeitlichen Verschiebung des Messverlaufs beider Messpunkte auf Grund der unterschiedlichen Position im System.

Bevorzugt werden die Druckmesswerte blutseitig an zwei Drucksensoren gemessen. Dabei ist es weiterhin bevorzugt, dass einer der beiden Drucksensoren (9) im arteriellen Zulauf, d.h. vor dem Dialysator und der andere Drucksensor (11) im venösen Ablauf, d.h. hinter dem Dialysator im Blutkreislauf der extrakorporalen Blutbehandlungseinheit angeordnet ist.

Die Ermittlung der Druckmesswerte erfolgt über Drucksensoren. Hierfür können die aus dem Stand der Technik bekannten Drucksensoren benutzt werden, wie z.B. piezoresistive, piezoelektrische, frequenzanaloge, Drucksensoren mit Hallelementen, kapazitive, induktive und/oder Kombinationen davon. Bevorzugt sind hierbei Drucksensoren, deren Abtastrate mindestens 20 Hz beträgt. Die Abtastrate bezeichnet hierbei die Rate mit der Druckmesswerte aus einem kontinuierlichen Signal entnommen werden.

Mit Beginn der Therapie wird dann mindestens ein Druckmesswert $P_x$, bevorzugt jedoch mindestens zwei Druckmesswerte bzw. mehrere Druckmesswerte an mehreren Drucksensoren gemessen. Wird an zwei Drucksensoren gemessen, so kann anstelle des Absolutdruckes die Druckdifferenz oder der Druckquotient in den erfindungsgemäßen Verfahren und Vorrichtungen verwendet werden. Sollte dann im Verlaufe der Dialysesitzung einer oder mehrere dieser Druckmesswerte, Druckdifferenzen oder Druckquotienten aus dem Toleranzbereich herausfallen bzw. den oberen Grenzwert überschreiten oder den unteren Grenzwert unterschreiten (je nachdem ob Absorbanz oder Transmission als Parameter verwendet wird), dann wird eine Verklottung angezeigt.

Hierzu kann während der Therapie aus einer vordefinierten Anzahl (n) gemessener Druckmesswerte ($P_x$) durch Extrapolation ein zukünftiger Druckmesswert ($P_{x+1(berechnet)}$) berechnet werden, worin x die Anzahl an Druckmessungen bezeichnet. Somit kann z.B. über die Bestimmung der letzten fünf Druckmesswerte ($P_x$, wobei x = 1 - 5, also über die Druckmesswerte $P_1$, $P_2$, $P_3$, $P_4$, $P_5$) der zukünftige Druckmesswert ($P_{x+1(berechnet)}$, wobei x = 5 ist, also Druckmesswert $P_6$) berechnet werden. Für diesen berechneten zukünftigen Druckmesswert ($P_{x+1(berechnet)}$) wird ein Toleranzbereich berechnet oder festgelegt. Somit bezeichnet x immer die Nummer der jeweiligen Druckmessung für die entsprechende Dialysebehandlung, $P_{10}$ steht also für den Druckmesswert der zehnten Messung im Verlauf der Dialysebehandlung. Das gleiche gilt analog für y, welches die Nummer der jeweiligen Absorbanzmessung bezeichnet. Die Variablen n und m stehen hingegen nicht für eine Nummer sondern für eine Anzahl, so dass n = 5 für eine Anzahl an n Messungen steht. Wird beispielsweise die Basislinie, also der Normdruck, mittels 600 Druckmessungen $P_x$ bestimmt, so ist n = 600.

Der Toleranzbereich begrenzt dabei den Bereich des Druckes nach oben und nach unten, der von dem gemessenen Druckmesswert ($P_{x+1(gemessen)}$) nicht über- oder unterschritten werden sollte bzw. dürfte. Dabei können die Bereiche, die nach oben bzw. nach unten nicht über- unterschritten werden dürfen, gleich groß sein, sie können aber auch unterschiedlich groß sein. So ist es bevorzugt, dass der Bereich nach oben hin größer ist als der Bereich nach unten. Liegt der dann tatsächlich gemessene Druckmesswert ($P_{x+1(gemessen)}$) innerhalb des Toleranzbereichs, dann ist keine Verklottung des Dialysators eingetreten und die Berechnung setzt sich entsprechend fort, indem nun erneut z.B. über die Bestimmung der letzten fünf Druckmesswerte ($P_x$, im vorliegenden Beispiel $P_2$, $P_3$, $P_4$, $P_5$, $P_6$) der zukünftige Druckmesswert ($P_{x+1(berechnet)}$, hier also $P_7$) und der dazugehörige Toleranzbereich berechnet wird. Die Bestimmung der letzten Druckmesswerte ($P_x$) erfolgt dabei bevorzugt nach dem FIFO (First-in-First-out) Prinzip, wonach der erste Druckmesswert der vorangegangenen Berechnung nun herausfällt und der zuletzt gemessene Druckmesswert ($P_{x+1}$) in die Berechnung mit einfließt. Somit wird erneut über die Bestimmung der letzten fünf gemessenen Druckmesswerte ($P_x$) der zukünftige Druckmesswert ($P_{x+1(berechnet)}$) und der dazugehörige Toleranzbereich berechnet.

Eine mögliche Ausführungsform umfasst, dass nicht zu Beginn der Dialysesitzung ein Toleranzbereich oder ein oberer und/oder ein unterer Grenzwert für alle zukünftigen Druckmesswerte festgelegt oder berechnet wird, sondern zu jedem einzelnen extrapolierten Druckmesswert die Grenzwertberechnung oder Toleranzbereichsberechnung erneut erfolgt.

Im Ergebnis führt dies dazu, dass vor jeder Messung des nächsten Druckmesswertes ($P_{x+1(gemessen)}$) zunächst ein zukünftiger Druckmesswert ($P_{x+1(berechnet)}$) aus den letzten gemessenen Druckmesswerten berechnet wird und für diesen zukünftigen Druckmesswert ($P_{x+1(berechnet)}$) weiterhin ein Toleranzbereich berechnet wird. Fällt nun der nächste gemessene Druckmesswert ($P_{x+1(gemessen)}$) aus diesem Toleranzbereich heraus ist das eine eindeutige Detektion der Verklottung, wenn zugleich der gemessene UV-Messwert ($A_{y+1(gemessen)}$) oder ($T_{y+1(gemessen)}$) außerhalb des Toleranzbereichs des berechneten UV-Messwertes ($A_{y+1(berechnet)}$) oder ($T_{y+1(berechnet)}$) liegt.

Anstelle eines Toleranzbereichs für den Druck, eines Toleranzbereichs für die UV-Transmission und eines Toleranzbereichs für die UV-Absorbanz kann auch ein oberer Grenzwert für den Druck, ein oberer Grenzwert für die UV-Transmission und ein unterer Grenzwert für die UV-Absorbanz berechnet werden. Dies kann nach den folgenden Formeln erfolgen.

Der **obere** Grenzwert für die gemessenen Druckmesswerte wird gemäß folgender Formel berechnet:

$$P_{Grenzwert} = P_{Basislinie} + P_{\Delta}$$

worin $P_{\Delta}$ als Funktion von Filtereigenschaften, einem prozentualen Anteil oder einem festen einstellbaren Offset des Drucks definiert wird.

Ebenso ist es aber auch möglich, dass zu Beginn oder während der Therapie ein fester Grenzwert festgelegt wird. Bei diesem festen oberen Grenzwert kann es sich beispielsweise um 30 mmHg (40 hPa), 40 mm Hg (53,3 hPa) oder 50 mm Hg (66,7 hPa) handeln oder der obere Grenzwert wird auf einen prozentualen Anteil der gemessenen Druckmesswerte bezogen. Wurden beispielsweise 10 Druckmesswerte gemessen, welche im Mittel 100 mm Hg (133,3 hPa) ergaben, so könnte der obere Grenzwert als eine 15%-Abweichung [oberer Grenzwert also 115 mm Hg (153,3 hPa), als eine 20%-Abweichung [oberer Grenzwert also 120 mm Hg (160,0 hPa), als eine 25%-Abweichung [oberer Grenzwert also 125 mm Hg (166,7 hPa) oder z.B. als eine 30%-Abweichung [oberer Grenzwert also 130 mm Hg (173,3 hPa) definiert werden. Es ist bevorzugt, wenn der Grenzwert des Drucks bezogen auf einen bestimmten Blutfluss mit einer prozentualen Abweichung von 1 angegeben wird, der Grenzwert also bei 10% liegt.

Der **untere** Grenzwert für die bestimmten UV-Absorbanzen wird beispielsweise gemäß folgender Formel berechnet:

$$A_{x+1} = F(A_1, \dots, A_n)$$

$$A_{Grenzwert\_x+1} = A_{x+1} + A_{\Delta}$$

worin $A_{\Delta}$ als Funktion von Filtereigenschaften, einem prozentualen Anteil oder einem festen, einstellbaren Offset der Absorbanz definiert wird.

Der obere Grenzwert für die Transmission wird analog dem oberen Grenzwert für die Absorbanz berechnet, da die Summe aus Absorbanz und Transmission der eingestrahlten UV-Intensität entspricht.

Der Toleranzbereich für die gemessenen UV-Absorbanzen wird beispielsweise als Bereich vom oberen Grenzwert für die UV-Absorbanz bis 5% unterhalb des extrapolierten Wertes festgelegt.

Weiterhin ist es möglich, nicht für jeden zu messenden nächsten Druckmesswert ($P_{x+1}$) einen zukünftigen Druckmesswert ($P_{x+1(berechnet)}$) und den dazugehörigen Toleranzbereich zu berechnen, sondern in bestimmten Intervallen, z.B. nur nach jedem zehnten Druckmesswert, einen zukünftigen Druckmesswert und den dazugehörigen Toleranzbereich zu berechnen. Dieses Intervall muss nicht gleichmäßig sein, sondern kann z.B. derart ausgestaltet sein, dass zu Beginn der Therapie ein größeres Intervall gewählt wird, welches im Laufe der Therapie kleiner wird.

In einer weiteren Ausführungsform ist es auch möglich, dass der Toleranzbereich nicht berechnet wird, sondern ein Toleranzbereich festgelegt wird, der für jeden gemessenen Druckmesswert gleichermaßen gilt. Solch ein festgelegter Toleranzbereich kann dadurch definiert sein, dass der gemessene Druckmesswert einen gewissen Druckwert nicht über - bzw. unterschreiten darf. Beispielsweise könnte festgelegt sein, dass jeder gemessene Druckmesswert einen Wert von 150 mmHg (entspricht 200 hPa) nicht überschreiten und einen Wert von 50 mmHg (entspricht 66,7 hPa) nicht unterschreiten darf. Dieser konkrete Toleranzbereich kann zu Beginn der Therapie festgelegt werden oder aber auch im Verlauf der Therapie verändert werden. Hierzu ist es auch möglich, auf die Daten von früheren Behandlungen eines Patienten zurückzugreifen und aus diesen Daten einen Toleranzbereich festzulegen.

In einer bevorzugten Ausführungsform wird die Überlegung genutzt, dass bei einem einwandfreien Dialysator die Druckmesswerte über den Verlauf der Behandlung - abgesehen von kleinen Schwankungen - für den eingestellten Blutfluss zumeist konstant sind. Im Prinzip sind die gemessenen Druckmesswerte vom jeweiligen Patienten, der Viskosität des Blutes und der Blutflussrate (BF) abhängig. Daraus ergibt sich, dass es möglich ist, zu Beginn der Dialyse für jeden Patienten für die eingestellte Blutflussrate eine Kalibrierung bzw. eine Feststellung des IST-Zustandes durchzuführen, welche die Druckmesswerte im Normalfall, d.h. ohne eine Verklottung darstellt. Daraus folgt, da die Druckmesswerte bei einem Dialysator ohne Verklottung für den jeweiligen Blutfluss annähernd konstant sind, dass zu Beginn der Therapie der Normalzustand der Druckmesswerte durch ein patientenspezifisches Basislevel aufgenommen wird. Dieser Basislevel ist von der Viskosität, dem Blutfluss und dem Dialysator abhängig. Sofern keine Verklottung auftritt, bleibt er bei einer konstanten Blutflussrate nahezu über die gesamte Therapie konstant. Eine Überwachung erfolgt daher durch Bewertung der Druckmesswerte bezogen auf eine zu Beginn berechnete Basislinie.

Der Normwert $P_{Basislinie}$ oder Basislevel des Drucks kann bei einer konstanten Blutflussrate $BF_z$ aus einem Mittelwert oder Median aus (n) gemessenen Druckmesswerten ($P_x$) bestimmt werden. Als Basislinie im Sinne dieser Erfindung dient eine Ausgleichsgerade, welche aus einer vordefinierten Anzahl (n) gemessener Druckmesswerte ($P_x$) berechnet wird. Hierzu sollten mindestens zwei Messungen durchgeführt werden, bevorzugt ist (n) > 10, weiter bevorzugt (n) > 100 und besonders bevorzugt ist (n) > 600. Es ist des Weiteren bevorzugt, wenn die (n) Druckmesswerte zu Anfang jeder Dialysebehandlung durchgeführt werden, also z.B. während der ersten Minute oder der ersten 5 Minuten. Dabei bezeichnet (n) die Anzahl der Messungen, welche zur Berechnung der Basislinie herangezogen werden. Danach kann die Bestimmung einer Basislinie in Intervallen wiederholt werden. Bevorzugt ist jedoch, wenn die Berechnung der Basislinie bei konstanter Blutflussrate nur zu Beginn einer Dialysebehandlung stattfindet. Die Druckmessung zur Berechnung einer Basislinie und die Berechnung der Basislinie muss aber wiederholt werden, falls der Blutfluss geändert wird.

Abweichungen von diesem Basislevel bzw. für diesen kalibrierten bzw. festgestellten normal Druckmesswert $P_{Basislinie\ von\ BFz}$ können nun dadurch erkannt werden, dass ein oberer Grenzwert berechnet wird, der die maximal erlaubte Änderung des Druckmesswertes definiert. Hierzu wird vor Beginn der Therapie von dem Anwender ein Eingabeparameter festgelegt, wobei dieser Eingabeparameter die Verklottung [%] oder Clearanceänderung [%] sein kann.

Dabei können die Grenzwerte oder Grenzen der Toleranzbereiche durch folgende Formel berechnet werden, wobei hier immer mit Bezug zum Basislevel nur eine relative Änderung betrachtet wird:

$$\text{oberer Grenzwert} = \text{Basislevel} + F\ (BF, Dialysator,[\%])$$

$$\text{unterer Grenzwert} = \text{Basislevel} - F\ (BF, Dialysator,[\%])$$

$$\text{Verklottung}[\%] = F(BF, Dialysator, \Delta P)$$

Die Parameter Blutfluss und Basislevel können zu Beginn der Therapie im System gemessen werden. Informationen über den Dialysatortyp können durch eine Ausmessung des Dialysatorvolumens mit Hilfe von Leitfähigkeitssonden erfolgen. Ebenso kann der Dialysatortyp durch manuelle Eingabe des Anwenders, RFID, Barcode Scanner oder mit einer Patientendiskette ermittelt werden.

Für die Drucküberwachung, d.h. die Vorausberechnung eines folgenden extrapolierten Druckmesswertes ist es daher bevorzugt, nicht eine Reihe unmittelbar vorher gemessener Druckmesswerte zu verwenden sondern die am Anfang der Dialysesitzung gemessenen Druckmesswerte zu verwenden, welche die Basislinie bilden, also eine im Idealfall waagerecht verlaufende Gerade und dann zu überprüfen, ob zukünftige Druckmesswerte von dieser Basisline (Baseline) über einen Toleranzbereich hinaus abweichen. Da die Basislinie von der Blutflussrate abhängig ist, wird immer dann eine neue Basislinie ermittelt, wenn die Blutflussrate geändert wird. Wird die Blutflussrate während der Dialysesitzung nicht verändert, so ist die Basislinie während der gesamten Dauer gültig, d.h. es ist nicht erforderlich, jedes Mal neue n gemessene Druckmesswerte ($P_x$) für die Berechnung des zukünftigen Druckmesswertes heranzuziehen, vielmehr kann auf die zur Ermittlung der Basislinie gemessenen n Druckmesswerte zurückgegriffen werden.

Die waagerecht verlaufende Gerade, welche die Basislinie bildet, entspricht einer Ausgleichsgeraden durch die anfänglich ermittelten n Druckmesswerte. Bei einer Auftragung des Drucks über die Zeit der Dialysebehandlung entspricht die Basislinie im Idealfall einer zur Zeitachse parallel verlaufenden Geraden durch den Mittelwert der zur Berechnung der Basslinie herangezogenen Druckmesswerte, zumindest für die Dauer des konstanten Blutflusses $BF_z$. Um diese Basislinie kann ein Toleranzbereich oder ein Toleranzband festgelegt werden. Dieser Toleranzbereich umfasst dabei den Bereich des Druckes nach oben und nach unten, der von dem gemessenen Druckmesswert (P$_{(gemessen)}$) nicht über- oder unterschritten werden sollte. Dabei können die Bereiche, die nach oben bzw. nach unten nicht über- oder unterschritten werden dürfen, gleich groß sein, sie können aber unterschiedlich groß sein. So ist es bevorzugt, dass der Bereich nach oben hin größer ist als der Bereich nach unten. Liegt der dann tatsächlich gemessene Druckmesswert (P$_{(gemessen)}$) innerhalb des Toleranzbereichs, dann ist keine Verklottung des Dialysators eingetreten und die Berechnung setzt sich entsprechend fort.

Der Ausdruck wie hierin verwendet, dass "aus einer vordefinierten Anzahl (n) gemessener Druckmesswerte (P$_x$) durch Extrapolation ein zukünftiger Druckmesswert (P$_{x+1(berechnet)}$) berechnet wird" bedeutet somit vorzugsweise, dass nicht durch Interpolationsmethoden ein zukünftiger Druckmesswert berechnet und dieser mit einem gemessenen Druckmesswert verglichen wird, sondern dass davon ausgegangen wird, dass der zukünftige Druckmesswert innerhalb eines Toleranzbereiches auf der Basislinie liegt, so dass es eigentlich keiner Berechnung der zukünftigen Druckmesswerte bedarf und anstelle der Berechnung die Annahme tritt, der zukünftige Druckmesswert läge innerhalb eines Toleranzbereiches auf der Basislinie. Liegt der gemessene Druckmesswert dann entgegen der Annahme außerhalb des Toleranzbereiches um die Basislinie, dann zeigt dieses Ergebnis Verklottung an, sofern es mit einer gemessenen UV-Absorbanz außerhalb des Toleranzbereichs für die berechnete UV-Absorbanz zusammenfällt.

Der Toleranzbereich für die gemessenen Druckmesswerte bzw. der Toleranzbereich um die Basislinie kann ein fester Grenzwert sein oder beispielsweise gemäß folgender Formel berechnet werden:

$$P_{Grenzwert} = P_{Basislinie\_von\_BFz} \pm P_{\Delta}$$

worin P$_{\Delta}$ als Funktion von Filtereigenschaften, einem prozentualen Anteil oder einem festen einstellbaren Offset des Drucks definiert wird.

Der Toleranzbereich könnte auch als ein fester Grenzbereich von Basislinie ± 30 mm Hg (40 hPa) oder Basislinie ± 40 mm Hg (53,3 hPa) oder Basislinie ± 50 mm Hg (66,7 hPa) definiert werden. Ferner könnte der Toleranzbereich als Basislinie ± 15% oder Basislinie ± 20% oder Basislinie ± 25% oder Basislinie ± 30% definiert werden.

Wird während der Dialysesitzung die Blutflussrate (BF) geändert, so sollte dies linear erfolgen. Danach wird eine neue Basislinie aus n Druckmesswerten ermittelt und danach verglichen, ob zukünftige Druckmesswerte innerhalb eines Toleranzbereichs um diese neue Basislinie liegen. Vorzugsweise sollte sicherheitshalber auch noch ein Vergleich der Basislinien vorgenommen werden, wobei gelten sollte:

$$BF1/BF2 = Basislinie1/Basislinie2$$

Somit ist es bei dem berechneten bzw. festgelegten oberen Grenzwert für die Druckmesswerte nicht notwendig eine Prädiktion des nächsten Druckmesswerts zu treffen, da zu Beginn der Therapie bereits ein Basislevel gemessen wurde. In dieser Ausführungsform wird der obere Grenzwert gemäß dem Eingabeparameter Verklottung [%] oder Clearanceänderung [%] berechnet, wobei zur Berechnung des oberen Grenzwerts mit Bezug zum Basislevel nur eine relative Änderung betrachtet wird.

Allgemein ist zu beachten, dass die Veränderung der Blutflussrate während der Therapie auch eine Veränderung der Druckmesswerte mit sich bringt. Daher ist es prinzipiell nach einer Änderung der Blutflussrate notwendig, eine erneute Kalibrierung bzw. Feststellung des IST-Zustandes durchzuführen, also eine neue Basislinie zu bestimmen.

Dies gilt in abgewandelter Form ebenso für den Fall, dass der Toleranzbereich durch eine Prädiktion des nächsten Druckmesswertes berechnet wird. Durch eine Veränderung der Blutflussrate während der Therapie tritt auch eine Veränderung der Druckmesswerte auf, die bei einer Prädiktion des nächsten Druckmesswertes aus den letzten gemessenen Druckmesswerten zu einem falsch-positiven Alarm führen würden. Daher ist nach einer Änderung der Blutflussrate kurzzeitig keine Prädiktion des nächsten Druckmesswertes möglich, da die zuletzt gemessenen Druckmesswerte noch mit einer veränderten Blutflussrate gemessen wurden. Sobald die Blutflussrate sich auf dem neuen Niveau eingestellt hat, kann auch wieder eine Prädiktion des nächsten Druckmesswertes und des dazugehörigen Toleranzbereiches berechnet werden, wobei der genaue Zeitpunkt davon abhängig ist, aus wie vielen zuletzt gemessenen Druckmesswerten der zukünftige Druckmesswert berechnet werden soll. In der Praxis ist der Zeitraum, in der keine Prädiktion des nächsten Druckmesswertes möglich ist, als vernachlässigbar einzustufen, da Drucksensoren bevorzugt sind, deren Abtastrate mindestens 20 Hz beträgt.

Problematisch ist in diesem Zusammenhang, dass eine Änderung der Druckmesswerte über den oberen Grenzwert bzw. über den Toleranzbereich hinaus auch andere Ursachen haben kann als Verklottung. Kommt es z.B. zu einem Knick im Schlauch oder der Bildung eines Koagels vor dem Dialysator, dann hat dies Auswirkungen auf die gemessenen Druckwerte, obwohl nicht eine Verklottung die Ursache ist. Daher bestehen einige Fälle, in denen die Messung der Druckmesswerte alleine zu einem falsch-positiven Ergebnis führen würde.

Daher wird die Druckwertmessung im Blutkreislauf erfindungsgemäß mit einer Messung der UV-Absorbanz mindestens einer harnpflichtigen Substanz im Dialysatausfluss der extrakorporalen Blutbehandlungseinheit kombiniert. Harnpflichtige Substanzen sind Stoffwechsel- oder Stoffwechselendprodukten, die zwingend mit dem Urin ausgeschieden werden. Darunter fallen beim Menschen vor allem Harnstoff, Harnsäure sowie Kreatinin.

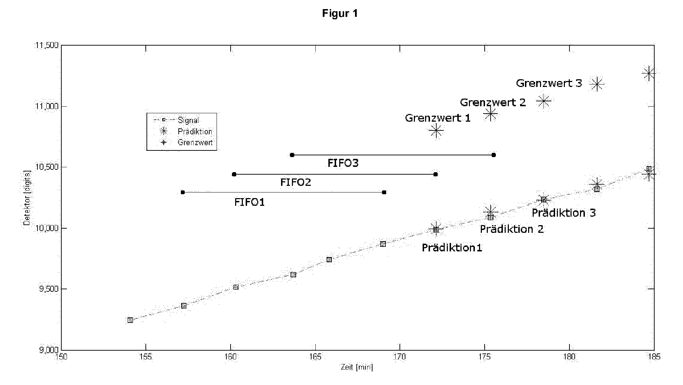

Gemessen wird mit UV-Sensoren, die auf dem Prinzip der Fotometrie beruhen und die UV-Absorbanz mindestens einer harnpflichtigen Substanz im Dialysatausfluss der Blutbehandlungseinheit messen. Durch die Messung einer Absorbanz A bei ca. $\lambda$=280nm wird der relative Anteil von harnpflichtigen Substanzen im Dialysat zu einem beliebigen Zeitpunkt während der Dialysetherapie bestimmt. Der Sensor selber befindet sich vorzugsweise im Abfluss hinter dem Dialysator und misst während der Therapie kontinuierlich die Absorbanz A im Abfluss (siehe hierzu auch Fig.1).

Zu Beginn wird der *Detektor*$_0$-Wert mit reiner Dialyseflüssigkeit ermittelt.

$$A = \log_{10}\left(\frac{Detektor_0}{Detektor_i}\right)$$

Während der Therapie wird der Wert Detektor$_i$ durch UV-Messungen bestimmt und nach oben genannter Formel die Absorbanz A berechnet. Für den Messablauf ist es zweckmäßig die Kalibrierung vor dem Anlegen des Patienten bzw. im Bypass durchzuführen.

Für die UV-Absorbanzmessung können handelsübliche Sensoren verwendet werden. In bevorzugten Modellen fließt der Flüssigkeitsstrom durch ein transparentes Ausflussteil, beispielsweise einen Schlauch, der durch eine zentrale Aussparung am UV-Sensor geführt wird. In anderen Worten, der UV-Sensor umgibt das Ausflussteil in einem Abschnitt ringförmig. Der UV-Strahl fällt hierbei durch das zentrale Segment des Ausflussteiles in seiner ganzen Breite. Dies führt zu einer Erhöhung der Messgenauigkeit, da Streuungs- und Beugungseffekte so reduziert werden. Bei der Auswahl des zumindest in diesem Abschnitt transparenten Wandmaterials des Ausflussteiles ist es vorteilhaft, wenn Materialien verwendet werden, bei denen nur eine geringe Lichtstreuung und Reflexion auftritt. Diese beiden Effekte wirken sich negativ auf die Messgenauigkeit aus. Insbesondere bei Messungen unterhalb von 200 nm Wellenlänge hat sich Quarzglas bewährt. Erfindungsgemäß kann dieses Glas auch in den Sensor eingebaut sein und auf der Wegstrecke durch den Sensor fließt die Dialyselösung frei durch dieses Glasrohr. Entsprechende Anschlüsse vor und nach dem Sensor herzustellen, liegt im Wissensbereich des durchschnittlichen Fachmanns auf diesem Gebiet. Die Fehlergenauigkeit des Messsystems sollte bevorzugt unter 15% liegen, bevorzugter unter 10% und am meisten bevorzugt unter 5%.

Aus Schutzgründen sollten, wenn aufgrund der Bauweise nötig, Sichtblenden um den Messbereich des UV-Sensors angebracht sein, um zu verhindern, dass menschliches Gewebe, vor allem aber die Augen des Personals und/oder der Patienten, mit dem UV-Messstrahl in Berührung kommt.

Zusätzlich zu der Druckmessung wird während der Therapie aus einer vordefinierten Anzahl (m) gemessener UV-Absorbanzwerte (A$_y$) durch Extrapolation ein zukünftiger UV-Absorbanzwert (A$_{y+1(berechnet)}$) berechnet, worin y die Anzahl an UV-Messungen bezeichnet. Somit kann z.B. über die Bestimmung der letzten fünf UV-Messungen (A$_y$, wobei y = 1 - 5) der zukünftige UV-Absorbanzwert (A$_{y+1(berechnet)}$, mit y = 5) berechnet werden. Für diesen berechneten zukünftigen UV-Absorbanzwert (A$_{y+1(berechnet)}$, mit y = 5) wird ein Toleranzbereich beispielsweise gemäß obiger Formel berechnet. Dieser Toleranzbereich begrenzt dabei den Bereich nach oben und nach unten, der von dem gemessenen UV-Absorbanzwert (A$_{y+1(gemessen)}$) nicht über- oder unterschritten werden sollte, weil dies ein Anzeichen für Verklottung sein könnte. Dabei können die Bereiche, die nach oben bzw. nach unten nicht überunterschritten werden dürfen, gleich groß sein, sie können aber auch unterschiedlich groß sein. So ist es zumeist bevorzugt, dass der Bereich nach unten hin größer ist, d.h. die Absorbanz nimmt ab, da weniger harnpflichtige Substanzen das UV-Licht absorbieren, als der Bereich nach oben hin.

Wurde der Toleranzbereich nicht über- bzw. unterschritten, dann ist keine Verklottung des Dialysators eingetreten und die Berechnung setzt sich entsprechend fort, indem nun erneut über die Bestimmung der letzten fünf UV-Absorbanzwerte (A$_y$) der zukünftige UV-Absorbanzwert (A$_{y+1(berechnet)}$) und der dazugehörige Toleranzbereich berechnet wird. Die Bestimmung der letzten UV-Absorbanzwerte (A$_y$) erfolgt dabei bevorzugt nach dem FIFO (First-in-First-out) Prinzip, wonach der erste UV-Absorbanzwert der vorangegangenen Berechnung nun herausfällt und der zuletzt gemessene UV-Absorbanzwert (A$_{y+1(gemessen)}$) in die Berechnung mit einfließt. Somit wird erneut über die Bestimmung der letzten fünf UV-Absorbanzwerte (A$_y$, mit y = 1 - 5) der zukünftige UV-Absorbanzwert (A$_{y+1(berechnet)}$, mit y = 5$_)$) und der dazugehörige Toleranzbereich berechnet.

Die Berechnung der zukünftigen UV-Absorbanz (A$_{y+1(berechnet)}$) mittels Extrapolation oder Interpolation kann auf verschiedene Weisen erfolgen, wobei bekannte Berechnungsmethoden eingesetzt werden können. Folgende Berechnungsmethoden können z.B. verwendet werden:

a) FIFO Fitting aus n Werten linear:

$$Det_{x+1} = F_{lin} (Det_1, \ldots, Det_n)$$

b) Fitting mittels einer e-Funktion:

$$A_{x+1} = F_{exp} (A_1, \ldots, A_n)$$

c) Vorausberechnung von $A_{x+1}$ mittels einer A0-Blutmessung des Patienten und Berechnung des Verlaufs auf Basis der Standard-Clearance und des Körpervolumens des Patienten (basierend auf 1. Kompartment-Modell):

$$A_{x+1} = F_{komplex} (A_1, \ldots, A_n, A_{oPatient}, Filter, \ldots)$$

Im Ergebnis führt dies dazu, dass vor jeder Messung des nächsten UV-Absorbanzwertes ($A_{y+1(gemessen)}$) zunächst ein zukünftiger UV-Absorbanzwert ($A_{y+1(berechnet)}$) aus den zuletzt gemessenen UV-Absorbanzwerten berechnet wird und für diesen zukünftigen UV-Absorbanzwert ($A_{y+1(berechnet)}$) weiterhin ein Toleranzbereich berechnet wird. Liegt nun der nächste gemessene UV-Absorbanzwert ($A_{y+1(gemessen)}$) außerhalb dieses Toleranzbereichs, ist das ein Anzeichen für Verklottung. Eine eindeutige Detektion der Verklottung erfolgt aber nur dann, wenn auch der gemessene Druckmesswert, der zeitgleich mit dem UV-Absorbanzwert ($A_{y+1(gemessen)}$) gemessen wurde, außerhalb des Toleranzbereichs für den Druckmesswert liegt.

Bevorzugt ist, dass nicht zu Beginn der Dialysesitzung ein Toleranzbereich oder ein oberer und/oder ein unterer Grenzwert für alle zukünftigen UV-Messwerte festgelegt oder berechnet wird, sondern zu jedem einzelnen extrapolierten UV-Messwert die Grenzwertberechnung oder Toleranzbereichberechnung erneut erfolgt.

Weiterhin ist es möglich, nicht für jeden zu messenden nächsten UV-Absorbanzwert ($A_{y+1(gemessen)}$) einen zukünftigen UV-Absorbanzwert ($A_{y+1(berechnet)}$) und den dazugehörigen Toleranzbereich zu berechnen, sondern in bestimmten Intervallen, z.B. nur nach jeder zehnten UV-Absorbanzmessung, einen zukünftigen UV-Absorbanzwert und den dazugehörigen Toleranzbereich zu berechnen. Dieses Intervall muss nicht gleichmäßig sein, sondern kann derart ausgestaltet sein, dass zu Beginn der Therapie ein größeres Intervall gewählt wird, welches im Laufe der Therapie kleiner wird.

In einer weiteren Ausführungsform ist es auch möglich, dass der Toleranzbereich nicht berechnet wird, sondern ein Toleranzbereich festgelegt wird, der für jeden gemessenen UV-Absorbanzwert gleichermaßen gilt. Solch ein festgelegter Toleranzbereich kann dadurch definiert sein, dass die gemessene UV-Absorbanz einen gewissen Wert nicht über - bzw. unterschreiten darf. Dieser konkrete Toleranzbereich kann zu Beginn der Therapie festgelegt werden oder aber auch im Verlauf der Therapie verändert werden. Hierzu ist es auch möglich, auf die Daten von früheren Behandlungen eines Patienten zurückzugreifen und aus diesen Daten einen Toleranzbereich festzulegen.

Die Messung der UV-Absorbanz erfolgt bevorzugt dahingehend, dass ein Detektor im Dialysatausfluss die Intensität der UV-Strahlung erfasst. Für den Fall, dass sich die Konzentration von harnpflichtigen Substanzen im Dialysatausfluss erhöht, also eine bessere Reinigungsleistung erzielt wird, erniedrigt sich die Intensität der UV-Strahlung, die am Detektor gemessen werden kann, da ein Teil der UV-Strahlung durch harnpflichtige Substanzen absorbiert wird. Für den umgekehrten Fall, ist eine Erhöhung der am Detektor gemessenen Intensität zu erwarten, da nun weniger harnpflichtige Substanzen im Dialysatausfluss vorliegen, welche die UV-Strahlung absorbieren können.

Während einer störungsfreien Therapie steigt die gemessene Intensität der UV-Strahlung am Detektor nahezu linear an und der Verlauf der gemessenen UV-Intensität hat den Verlauf einer exponentiellen Funktion bzw. doppelt exponentiellen Funktion.

Sofern aber die Reinigungswirkung des Dialysators durch Verklottung nachlässt, führt dies direkt proportional zu einem weiteren Anstieg der gemessenen UV-Intensität, da dem Patienten nun weniger harnpflichtige Substanzen entzogen werden und damit auch weniger harnpflichtige Substanzen im Dialysatausfluss vorhanden sind.

In einer weiteren Ausführungsform wird während der Therapie aus einer vordefinierten Anzahl (m) gemessener UV-Absorbanzwerte ($A_y$) durch Extrapolation ein zukünftiger UV-Absorbanzwert ($A_{y+1(berechnet)}$) berechnet, worin y die Anzahl an UV-Messungen bezeichnet. Somit kann z.B. über die Bestimmung der letzten fünf UV-Messungen ($A_y$, wobei y = 1 - 5 ist) der zukünftige UV-Absorbanzwert ($A_{y+1(berechnet)}$, mit y = 5) berechnet werden. Für diesen berechneten zukünftigen UV-Absorbanzwert ($A_{y+1(berechnet)}$, mit y = 5) wird ein unterer Grenzwert beispielsweise gemäß obiger Formel berechnet. Dieser untere Grenzwert begrenzt dabei den Bereich nach unten, der von dem gemessenen UV-Absorbanzwert ($A_{y+1}$) nicht unterschritten werden soll, da dies bedeutet, dass weniger harnpflichtige Substanzen das UV-Licht absorbieren.

Wurde der untere Grenzwert nicht unterschritten, dann ist keine Verklottung des Dialysators eingetreten und die Berechnung setzt sich entsprechend fort, indem nun erneut über die Bestimmung der letzten fünf UV-Absorbanzwerte ($A_y$) der zukünftige UV-Absorbanzwert ($A_{y+1(berechnet)}$) und der dazugehörige untere Grenzwert berechnet wird. Die Bestimmung der letzten UV-Absorbanzwerte ($A_y$) erfolgt dabei bevorzugt nach dem FIFO (First-in-First-out) Prinzip, wonach der erste UV-Absorbanzwert der vorangegangenen Berechnung nun herausfällt und der zuletzt gemessenen UV-Absorbanzwert ($A_{y+1(gemessen)}$) in die Berechnung mit einfließt. Somit wird erneut über die Bestimmung der letzten fünf UV-Absorbanzwerte ($A_y$, wobei y = 1 - 5) der zukünftige UV-Absorbanzwert ($A_{y+1(berechnet)}$, mit y = $_5$) und der dazugehörige untere Grenzwert berechnet.

Im Ergebnis führt dies dazu, dass vor jeder Messung des nächsten UV-Absorbanzwertes ($A_{y+1(gemessen)}$) zunächst ein zukünftiger UV-Absorbanzwert ($A_{y+1(berechnet)}$) aus den zuletzt gemessenen UV-Absorbanzwerten berechnet wird und für diesen zukünftigen UV-Absorbanzwert ($A_{y+1(berechnet)}$) weiterhin ein unterer Grenzwert berechnet wird. Unterschreitet nun der nächste gemessene UV-Absorbanzwert ($A_{y+1(gemessen)}$) diesen unteren Grenzwert ist das ein Anzeichen für Verklottung.

In einer weiteren Ausführungsform wird die UV-Absorbanz mindestens einer harnpflichtigen Substanz durch Messung der Transmission der eingestrahlten UV-Strahlung bestimmt und ein oberer Grenzwert für die gemessene Transmission berechnet. Aus einer vordefinierten Anzahl (m) gemessener Transmissionen ($T_y$) wird durch Extrapolation eine zukünftige UV-Transmission ($T_{y+1(berechnet)}$) berechnet, worin y die Anzahl an UV-Messungen bezeichnet. Somit kann z.B. über die Bestimmung der letzten fünf UV-Messungen ($T_y$, wobei y = 1 - 5 ist) der zukünftige Transmissionswert ($T_{y+1(berechnet)}$, mit y = $5$) berechnet werden. Für diesen berechneten zukünftigen Transmissionswert ($T_{y+1(berechnet)}$, mit y = 5) wird ein oberer Grenzwert z.B. gemäß der hierin angegebenen Formel berechnet. Dieser obere Grenzwert begrenzt dabei den Bereich nach oben, der von dem gemessenen Transmissionswert ($T_{y+1(gemessen)}$) nicht überschritten werden soll, da dies bedeutet, dass weniger harnpflichtige Substanzen das UV-Licht absorbieren.

Auch in diesem Fall erfolgt die Messung der UV-Transmission bevorzugter Weise dahingehend, dass ein Detektor im Dialysatausfluss die Intensität der UV-Strahlung erfasst. Für den Fall, dass sich die Konzentration von harnpflichtigen Substanzen im Dialysatausfluss erhöht, also eine bessere Reinigungsleistung erzielt wird, erniedrigt sich auch die UV-Transmission, die am Detektor gemessen werden kann, da ein Teil der UV-Strahlung durch harnpflichtige Substanzen absorbiert wird. Für den umgekehrten Fall, ist eine Erhöhung der am Detektor gemessenen Transmission zu erwarten, da nun weniger harnpflichtige Substanzen im Dialysatausfluss vorliegen, welche die UV-Strahlung absorbieren können.

Während einer störungsfreien Therapie steigt die gemessene Transmission der UV-Strahlung am Detektor nahezu linear an und der Verlauf der gemessenen UV-Transmission hat den Verlauf einer exponentialen Funktion bzw. doppelt exponentialen Funktion.

Sofern aber die Reinigungswirkung des Dialysators durch Verklottung nachlässt, führt dies direkt proportional zu einem weiteren Anstieg der gemessenen UV-Transmission, da dem Patienten nun weniger harnpflichtige Substanzen entzogen werden und damit auch weniger harnpflichtige Substanzen im Dialysatausfluss vorhanden sind.

Durch eine Veränderung der Blutflussrate während der Therapie tritt auch eine Veränderung der UV-Absorbanz bzw. UV-Transmission auf, die bei einer Prädiktion der nächsten UV-Absorbanz bzw. UV-Transmission aus den letzten gemessenen UV-Absorbanz bzw. UV-Transmission Messwerten zu einem falsch-positiven Alarm führen würden. Daher ist nach einer Änderung der Blutflussrate kurzzeitig keine Prädiktion der nächsten UV-Absorbanz bzw. UV-Transmission möglich, da die zuletzt gemessenen UV-Absorbanzen bzw. UV-Transmissionen noch mit einer veränderten Blutflussrate gemessen wurden. Sobald die Blutflussrate sich auf dem neuen Niveau eingestellt hat, kann auch wieder eine Prädiktion der nächsten UV-Absorbanz bzw. UV-Transmission und des dazugehörigen Toleranzbereiches bzw. Grenzwertes berechnet werden, wobei der genaue Zeitpunkt davon abhängig ist, aus wie vielen zuletzt gemessenen UV-Absorbanzen bzw. UV-Transmissionen der zukünftige UV-Absorbanzwert bzw. UV-Transmissionswert berechnet werden soll.

Die Definition der Grenzwerte erfolgt durch einen, von dem Anwender festgelegten Eingabeparameter, wobei dieser Eingabeparameter die Verklottung [%] oder Clearanceänderung [%] sein kann.

Anhand dieses Eingabeparameters und durch zwei Übertragungsfunktionen wird der Grenzwert für den oder die Druckmesswerte berechnet.

Wurde als Anwendereingabe die Verklottungsgrenze [%] definiert, so gilt, die Verklottung ist proportional zur Clearance. Mit Hilfe der Prädiktion bzw. des Sollverlaufs kann so der Grenzwert für den nächsten Messwert bestimmt werden.

(4.1)

$$A_{Dialysat+n+1} = \left( Linearisierung\left( \sum_{i=1}^{5} A_{Dialysat\_n-i} \right) \right) \cdot (n+1)$$

(4.2)

$$A_{Dialysat+1} = \left( \frac{A_{Blut+1} \cdot Clearance}{Dialyatfluss} \right)$$

(4.3)

$$A_{Blut+1\_Grenze} = A_{Dialysat+1} \cdot Faktor\_DFK \cdot \left( \frac{1}{1 + Verklottung[\%]} \right)$$

(4.4)

$$\frac{Dialyatfluss}{Clearance} = Faktor\_DFC$$

(4.5)

$$\left( A_{Blut+1\_Grenze} \right) = A_{Blut+1\_Grenze} \cdot \frac{1}{(1 + Verklottung[\%])}$$

A steht dabei für die Absorbanz des Signals, welches proportional zur Konzentration ist. Da der Faktor_DFC konstant ist, kann die Annahme getroffen werden, dass der Verlauf der Absorbanz auf der Dialysatseite proportional zum Verlauf der Absorbanz auf der Blutseite ist (4.3). Somit kann der Grenzwert einer Verklottung [%] durch die Formel 4.5 berechnet werden.

Auch bei der Messung der UV-Absorbanz bzw. UV-Transmission stellen sich ähnliche Probleme, wie bei der Messung von Druckwerten, da Änderungen über den Grenzwert bzw. über den Toleranzbereich hinaus auch andere Ursachen haben können, die nicht direkt auf eine Verklottung des Dialysators zurückzuführen sind. Bildet sich beispielsweise ein Koagel hinter dem Dialysator, dann hat dies Auswirkungen auf die gemessene UV-Absorbanz bzw. UV-Transmission, obwohl hierfür nicht die Verklottung die Ursache ist. Daher bestehen einige Fälle, in denen die Messung der UV-Absorbanz bzw. UV-Transmission alleine zu einem falsch-positiven Ergebnis führen würde.

Erst durch die Kombination beider Messmethoden kann eine sichere Aussage darüber getroffen werden, ob eine Verklottung des Dialysators eingetreten ist. Während der Therapie wird sowohl die UV-Absorbanz bzw. UV-Transmission an der UV-Messeinrichtung als auch mindestens ein Druckmesswert im Blutkreislauf gemessen und überwacht. Die Überwachung umfasst dabei die Berechnung und/oder Festlegung von Grenzwerten oder Toleranzbereichen, wodurch Varianzen im normalen Verlauf kompensiert werden, die Wahrscheinlichkeit von Fehlalarmen deutlich reduziert wird und die Verklottung als solche erkannt werden kann.

Bei einem Abknicken eines Schlauchs und somit höheren Durchflusswiederstands würde es zu einem Druckanstieg kommen, der jedoch auf Grund des konstanten Blutflusses keine Auswirkung auf die Dialyseeffektivität hätte.

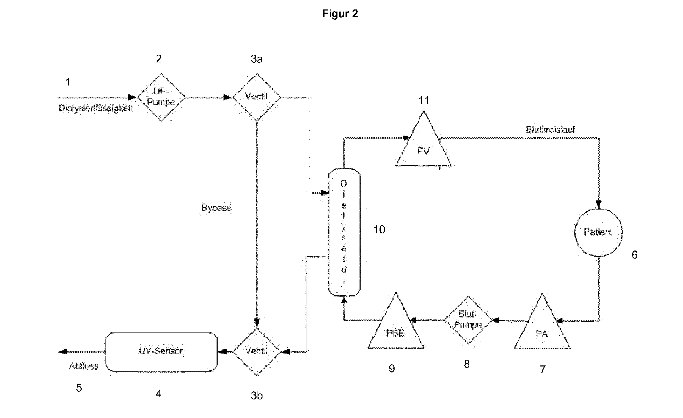

Die Figuren 1 und 2 veranschaulichen das Prinzip der Erfindung. In der Figur 1 ist beispielhaft ein Verlauf der UV-Transmission während einer Dialyse Therapie dargestellt. Für den oberen Grenzwert 1 werden die Messwerte der UV-Transmission der Minuten 157-169 herangezogen. Mit Hilfe der extrapolierten Messwerte wird das Signal "Prädiktion 1" für die nächste Messung in Minute 172 berechnet. Aus Prädiktion 1 wird der obere Grenzwert 1 berechnet und später mit der gemessenen UV-Transmission in Minute 172 verglichen. Für die Berechnung des oberen Grenzwertes wird eine Anwendereingabe (z.B. Verklottungsgrenze [%]) benötigt. Die Übertragungsfunktion benötigt weiterhin den Blutfluss und den Dialysatortypen. Sollte der Messwert oberhalb oder auf dem oberen Grenzwert 1 liegen ist eine geringere Reinigungswirkung des Dialysators die Ursache.

Fig.2 zeigt den Aufbau zur Erkennung von Verklottung mit Hilfe eines UV-Sensors und den Drucksensoren (9, 11) während einer Dialysebehandlung. Die Dialysierflüssigkeit (1) wird in der Dialysemaschine über die Dialysatflusspumpe (2) zum Dialysator (10) gepumpt. Dort kommt sie mit dem ungereinigten Blut des Patienten über eine halbdurchlässige Membran in Kontakt, so dass durch diffusive und konvektive Transportmechanismen ein Stoffaustausch stattfindet. Die nun mit harnpflichtigen Substanzen belastete Dialysierflüssigkeit passiert nach Verlassen des Dialysators (10) den UV-Sensor (4), welcher die Absorbanz der Flüssigkeit bei ca. 280nm misst. Danach wird das verunreinigte Dialysat dem Abfluss (5) zugeführt. Durch das gleichzeitige Schalten der Ventile (3a) und (3b) kann das frische Dialysat am Dialysator vorbeigeführt werden, um so den UV-Sensor zu kalibrieren. Dem Dialysepatient (6) wird das zu reinigende Blut arteriell entzogen. Der arterielle Zulaufdrucksensor (7) überwacht die Druckverhältnisse am arteriellen Zugang. Das Blut wird mit einer Blutpumpe (8) zum Dialysator (10) gefördert. Ein arterieller Einlaufdrucksensor (9) ist dem Dialysator (10) vorgeschaltet. Im Dialysator (10) werden dem Blut harnpflichtige Substanzen entzogen. Nach dem Passieren des Dialysators (10) wird das gereinigte Blut dem Patienten zurückgegeben. Der venöse Drucksensor (11) überwacht den Druck am venösen Patientenzugang.

Weiterhin umfasst die vorliegende Erfindung auch eine Vorrichtung zur extrakorporalen Blutbehandlung mit der das erfindungsgemäße Verfahren durchgeführt werden kann.

Dabei umfasst eine erfindungsgemäße Vorrichtung zur extrakorporalen Reinigung von Blut:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte Toleranzbereiche um die Basislinie und für die gemessenen Absorbanzen hinterlegt sind (es ist mindestens ein festgelegter Toleranzbereich für die Basislinie und mindestens ein festgelegter Toleranzbereich für die gemessenen Absorbanzen hinterlegt, d.h. gespeichert),
- eine Datenverarbeitungseinheit, welche aus einer vordefinierten Anzahl (n) gemessener Druckmesswerte ($P_x$), worin x die Anzahl an Druckmessungen bezeichnet, eine als Basisilinie bezeichnete Ausgleichsgerade berechnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{(gemessen)}$) innerhalb des festgelegten Toleranzbereichs um die Basislinie liegt und ob die gemessene UV-Absorbanz ($A_{y+1(gemessen)}$) innerhalb des festgelegten Toleranzbereichs für die berechnete UV-Absorbanz ($A_{y+1(berechnet)}$) liegt, wobei Verklottung detektiert und angezeigt wird, wenn der gemessene Druckmesswert ($P_{(gemessen)}$) außerhalb des festgelegten Toleranzbereichs um die Basislinie liegt und die gemessene UV-Absorbanz ($A_{y+1(gemessen)}$) außerhalb des festgelegten Toleranzbereichs für die berechnete UV-Absorbanz ($A_{y+1(berechnet)}$) liegt.

Weiterhin umfasst die vorliegende Erfindung eine Vorrichtung zur extrakorporalen Reinigung von Blut, welche aufweist:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einem Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen über der Basislinie und festgelegte untere Grenzen für die gemessenen Absorbanzen hinterlegt sind,
- eine Datenverarbeitungseinheit, welche aus einer vordefinierten Anzahl (n) gemessener Druckmesswerte ($P_x$),

worin x die Anzahl an Druckmessungen bezeichnet eine als Basisilinie bezeichnete Ausgleichsgerade berechnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{(gemessen)}$) den Grenzwert über der Basislinie übersteigt und ob die gemessene UV-Absorbanz ($A_{y+1(gemessen)}$) den Grenzwert für die berechnete UV-Absorbanz ($A_{y+1(berechnet)}$) unterschreitet, wobei Verklottung detektiert und angezeigt wird, wenn der gemessene Druckmesswert ($P_{(gemessen)}$) den Grenzwert über der Basislinie übersteigt und die gemessene UV-Absorbanz ($A_{y+1(gemessen)}$) den Grenzwert für die berechnete UV-Absorbanz ($A_{y+1(berechnet)}$) unterschreitet.

Zudem bezieht sich die Erfindung auf eine Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Transmission T der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen für die gemessenen Druckmesswerte und für die gemessenen Transmissionen hinterlegt sind,
- eine Datenverarbeitungseinheit, welche aus einer vordefinierten Anzahl (n) gemessener Druckmesswerte ($P_x$), worin x die Anzahl an Druckmessungen bezeichnet, eine als Basisilinie bezeichnete Ausgleichsgerade berechnet und aus einer vordefinierten Anzahl (m) gemessener Transmissionen ($T_y$) durch Extrapolation eine zukünftige Transmission ($T_{y+1(berechnet)}$) berechnet, worin y die Anzahl an UV-Messungen bezeichnet und bestimmt, ob der gemessene Druckmesswert ($P_{(gemessen)}$) den Grenzwert über der Basislinie übersteigt und ob die gemessene Transmission ($T_{y+1(gemessen)}$) den Grenzwert für die berechnete Transmission ($T_{y+1(berechnet)}$) übersteigt, wobei Verklottung detektiert und angezeigt wird, wenn der gemessene Druckmesswert ($P_{(gemessen)}$) den Grenzwert über der Basislinie übersteigt und die gemessene Transmission ($T_{y+1(gemessen)}$) den Grenzwert für die berechnete Transmission ($T_{y+1(berechnet)}$) übersteigt.

Ferner betrifft die Erfindung eine Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte Toleranzbereiche für die gemessenen Druckmesswerte und festgelegte Toleranzbereiche für die gemessenen Absorbanzen hinterlegt sind,
- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{x+1(gemessen)}$) innerhalb des festgelegten Toleranzbereichs für den berechneten Druckmesswert ($P_{x+1(berechnet)}$) liegt und ob die gemessene UV-Absorbanz ($A_{y+1(gemessen)}$) innerhalb des festgelegten Toleranzbereichs für die berechnete UV-Absorbanz ($A_{y+1(berechnet)}$) liegt, wobei Verklottung detektiert und angezeigt wird, wenn der gemessene Druckmesswert ($P_{x+1(gemessen)}$) außerhalb des festgelegten Toleranzbereichs für den berechneten Druckmesswert ($P_{x+1(berechnet)}$) liegt und die gemessene UV-Absorbanz ($A_{y+1(gemessen)}$) außerhalb des festgelegten Toleranzbereichs für die berechnete UV-Absorbanz ($A_{y+1(berechnet)}$) liegt.

Weiterhin umfasst die vorliegende Erfindung eine Vorrichtung zur extrakorporalen Reinigung von Blut:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen für die gemessenen Druckmesswerte und festgelegte untere Grenzen für die gemessenen Absorbanzen hinterlegt sind,
- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{x+1(gemessen)}$) den Grenzwert für den berechneten Druckmesswert ($P_{x+1(berechnet)}$) übersteigt und ob die gemessene UV-Absorbanz ($A_{y+1(gemessen)}$) den Grenzwert für die berechnete UV-Absorbanz ($A_{y+1(berechnet)}$) unterschreitet, wobei Verklottung detektiert und angezeigt wird, wenn der gemessene Druckmesswert ($P_{x+1(gemessen)}$) den Grenzwert für den berechneten Druckmesswert ($P_{x+1(berechnet)}$) übersteigt und die gemessene UV-Absorbanz ($A_{y+1(gemessen)}$) den Grenzwert für die berechnete UV-Absorbanz ($A_{y+1(berechnet)}$) unterschreitet.

Ebenso umfasst die vorliegende Erfindung eine Vorrichtung zur extrakorporalen Reinigung von Blut:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Transmission T der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen für die gemessenen Druckmesswerte und festgelegte obere Grenzen für die gemessenen Transmissionen hinterlegt sind,
- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener Transmissionen ($T_y$) durch Extrapolation eine zukünftige Transmission ($T_{y+1(berechnet)}$) berechnet, worin y die Anzahl an UV-Messungen bezeichnet und bestimmt, ob der gemessene Druckmesswert ($P_{x+1(gemessen)}$) den Grenzwert für den berechneten Druckmesswert ($P_{x+1(berechnet)}$) übersteigt und ob die gemessene Transmission ($T_{y+1(gemessen)}$) den Grenzwert für die berechnete Transmission ($T_{y+1(berechnet)}$) übersteigt, wobei Verklottung detektiert und angezeigt wird, wenn der gemessene Druckmesswert ($P_{x+1(gemessen)}$) den Grenzwert für den berechneten Druckmesswert ($P_{x+1(berechnet)}$) übersteigt und die gemessene Transmission ($T_{y+1(gemessen)}$) den Grenzwert für die berechnete Transmission ($T_{y+1(berechnet)}$) übersteigt.

Bei dem Dialysator handelt es sich vorzugsweise um einen Kapillardialysator. Der Begriff Kammer ist dabei nicht wörtlich zu verstehen, sondern beschreibt den Umstand, dass z.B. die Kapillaren von Dialysierflüssigkeit umflossen werden, was der ersten Kammer entspricht und das Blut in den Kapillaren fließt, also der zweiten Kammer.

Die obigen Vorrichtungen zur extrakorporalen Reinigung von Blut umfassen des Weiteren vorzugsweise ein Mittel zur Bestimmung der Blutflussrate $Q_B$ in der Blutzuführleistung und/oder der Blutabführleitung.

Ferner dient der Eingangsdrucksensor (PBE) vor dem Dialysator dazu, den Eingangsdruck in den Dialysator zu messen und der Ausgangsdruckmesser (PV) nach dem Dialysator dazu, den Ausgangsdruck aus dem Dialysator zu messen.

**Referenzzeichenliste**:

| | |
|---|---|
| 1 | Dialysierflüssigkeit |
| 2 | Dialysatflusspumpe |
| 3a/b | Ventil |
| 4 | UV-Detektor |
| 5 | Abfluss Dialysierflüssigkeit |
| 6 | Patient |
| 7 | Arterieller Zulaufdruckmesser |
| 8 | Blutpumpe |
| 9 | Arterieller Einlaufdrucksensor (PBE) |
| 10 | Dialysator |
| 11 | Venöser Drucksensor (PV) |

**Figurenbeschreibung**

**Figur 1**

Verlauf der gemessenen UV-Transmission während einer Dialyse Therapie.

Auf der y-Achse ist die gemessene UV-Transmission [digits] aufgetragen und auf der x-Achse die Zeit [min]. Alle drei Minuten wurde ein Messpunkt aufgenommen. Anhand der vorangegangenen Messwerte werden die zukünftigen Messwerte "Prädiktion 1, 2, 3" berechnet. Auf dieser Basis erfolgt weiterhin die Berechnung der dazugehörigen oberen "Grenzwerte 1, 2, 3". Die Bestimmung der vorangegangenen Messwerte erfolgt dabei nach dem FIFO (First-in-First-out) Prinzip, wonach der erste Messwert der vorangegangenen Berechnung nun herausfällt. Dies wird durch die Balken "FIFO 1, 2, 3" verdeutlicht, welche die jeweiligen Messwerte umfassen, die für die Berechnung des zukünftigen Messwertes "Prädiktion 1, 2, 3" und dem dazugehörigen oberen "Grenzwert 1, 2, 3" als Grundlage dienen.

**Figur 2**

Flussdiagramm zur Darstellung der Position des UV-Sensors.

**Figur 3**

Verlauf des Detektorsignals des UV-Sensors während einer laufenden Therapie. Auf der x-Achse ist die Zeit, d.h. die Dauer der Dialysesitzung aufgetragen. Auf der Y-Achse ist die am UV-Detektor gemessene UV-Intensität aufgetragen, d.h. die Intensität der UV-Strahlung, welche die Messstrecke passiert hat und nicht absorbiert worden ist. Zu Beginn der Messung, d.h. am Anfang der Dialysesitzung wird das Dialysat am stärksten mit der zu messenden UV-aktiven harnpflichtigen Substanz beladen, so dass die Absorbanz hoch und die gemessene UV-Intensität dementsprechend gering ist. Je mehr zu messende UV-aktive harnpflichtige Substanz dem Blut entzogen wird, desto weniger kann noch in die Dialysierflüssigkeit übergehen, so dass die Konzentration an zu messender UV-aktiver harnpflichtiger Substanz im Dialysatausfluss abnimmt und damit die Absorbanz sinkt und die Transmission steigt. Dieser Anstieg der UV-Intensität ist nahezu linear, sofern keine Verklottung auftritt. Ein starker Anstieg der UV-Intensität wie z.B. bei ca. 200 Minuten gezeigt deutet auf eine Verklottung hin, wobei auch andere Ursachen, wie z.B. die Herabsetzung der Blutflussrate zu einem solchen Anstieg hätten führen können. Ein derartiger Anstieg der UV-Intensität alleine, erlaubt daher noch keine eindeutige Aussage, dass eine Verklottung vorliegt.

**Figur 4**

Verlauf der Druckmessung während einer laufenden Therapie

Auf der x-Achse ist die Zeit, d.h. die Dauer der Dialysesitzung aufgetragen. Auf der Y-Achse ist der Druck am Drucksensor (9) [mmHg] aufgetragen. Bei einem einwandfreien Dialysator sind die Druckmesswerte über den Verlauf der Behandlung - abgesehen von kleinen Schwankungen - für den eingestellten Blutfluss konstant. Erst durch die Verklottung kommt es zu einem erhöhten Durchflusswiderstand, durch den der Druck am Drucksensor (9) zunimmt. Der Balken repräsentiert dabei einen voreingestellten Grenzwert von +50 mmHg (66,7 hPa).

**Figur 5**

Prädiktion des Sollverlaufs des Detektorsignals durch eine Messung der letzten fünf Messwerte.

Auf der x-Achse ist die Zeit, d.h. die Dauer der Dialysesitzung aufgetragen. Auf der y-Achse ist die am UV-Detektor gemessene UV-Intensität aufgetragen, d.h. die Intensität der UV-Strahlung, welche die Messstrecke passiert hat und nicht absorbiert worden ist. Alle drei Minuten wurde ein Messwert aufgenommen. Aus den letzten fünf Messwerten der gemessenen UV-Intensität an der UV-Messeinrichtung im Dialysatausfluss ergibt sich, dass das Messsignal im Mittel mit ca. 43,6 Detektordigits/min ansteigt.

**Beispiele**

**Beispiel 1**

Während einer Dialysebehandlung wurde die Verklottung des Dialysators durch Messung der UV-Absorbanz von Harnsäure im Dialysatausfluss und Messung der Drücke an den Drucksensoren (9, 11) überwacht. Die Verklottungsgrenze wurde mit 10% festgelegt.

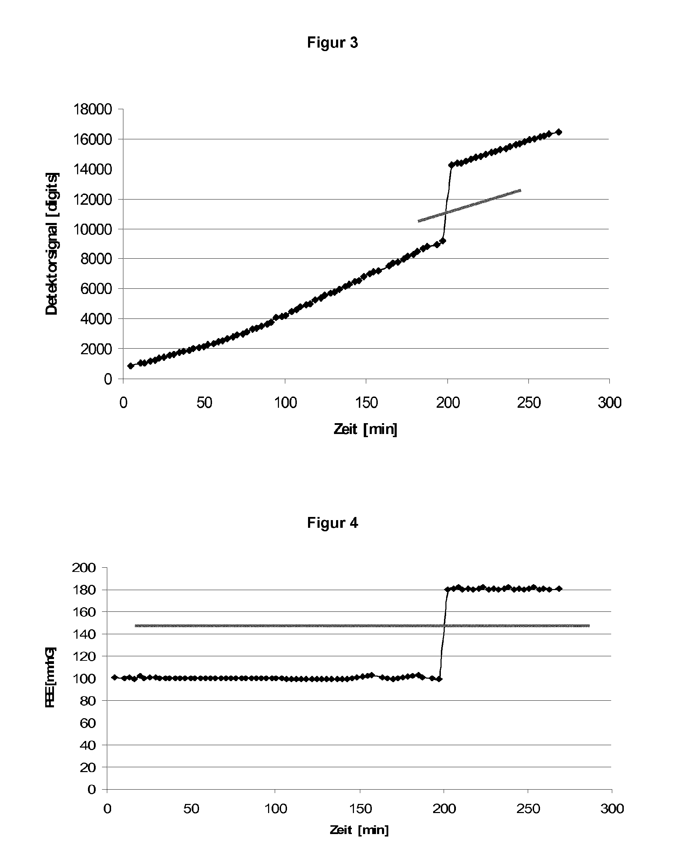

Die Fig.3 zeigt den Verlauf der gemessenen UV-Intensität am UV-Detektor während einer laufenden Therapie. Während einer störungsfreien Therapie steigt die gemessene UV-Intensität am UV-Detektor nahezu linear an und hat den Verlauf einer exponentiellen Funktion bzw. doppelt exponentiellen Funktion.

Nach knapp 200 Minuten der Behandlung trat eine Verklottung des Dialysators auf, wodurch eine sofortige Verringerung der Clearance verursacht wurde. Die Verringerung der Clearance in Fig.3 ist deutlich daran zu erkennen, dass weniger Harnsäure im Dialysatausfluss vorhanden ist und daher abrupt eine höhere UV-Intensität am UV-Detektor gemessen wird. Der eingezeichnete Balken repräsentiert dabei die berechneten Grenzwerte für die Messwerte zu den jeweiligen Zeitpunkten.

Die Grenzwerte wurden berechnet, in dem zuerst der Sollverlauf des Detektorsignals durch eine FIFO Messung über die letzten fünf Messwerte bestimmt wurde. Die

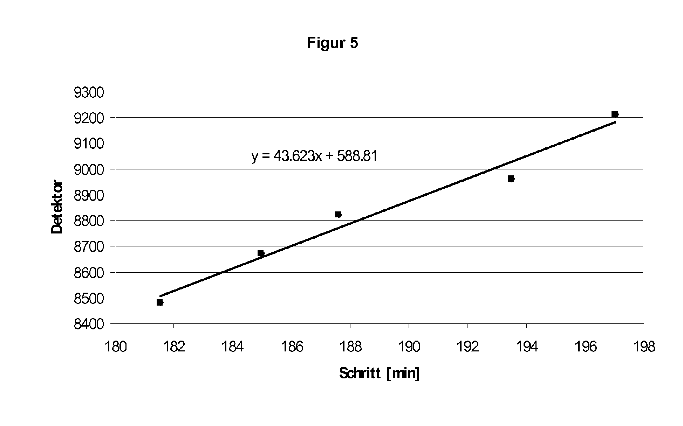

Fig.5 zeigt die letzten fünf Messwerte der gemessenen UV-Intensität am UV-Detektor im Dialysatausfluss, wobei alle drei Minuten ein Messwert aufgenommen wurde. Vor der Verklottung stieg das Detektorsignal im Mittel mit ca. 43,6 Detektordigits/min an. Die Prädiktion ergab, dass der nächste Messpunkt bei ca. 9343 digits liegen müsste.

Digit oder auch als Detektorwert bezeichnet ist das Signal des Analog-Digital Wandlers, in vorliegenden Fall mit einem 16Bit Bereich von 0 bis 65535. Dieser Wert des $\mu$C wird bei Wasser auf 55000 (Detektor$_{Wasser}$) geregelt (Absorbanz = 0). Basierend darauf kann der Verlauf des Signal am UV-Sensor entweder durch die Absorbanz exponentiell betrachtet werden oder linear anhand der Detektorwerte (in digits). Detektor$_{aktuell}$ ist der jeweils gemessene Wert während der Behandlung.

$$Absorbanz_i = log\left(\frac{Detektor_{Wasser}}{Detektor_{aktuell}}\right)$$

Bei einer Verklottungsgrenze von 10% ergab dies folgenden Grenzwert:

$$Detektor_{min=200} = 9213 + (43,6*3min) = 9343,8 \text{ digits}$$

$$A_{min=200} = log(55000/9343,8) = 0,769$$

$$A_{min=200\ Grenzwert} = 0,769/1,1 = 0,699$$

$$Detektor_{Grenzwert\_min200} = (55000/10^{0,669}) = 10977 \text{ digits}$$

Mit einem gemessenen Detektorsignal von weit über 14000 ist der Grenzwert zum Zeitpunkt 200 Minuten der Behandlung überschritten.

Durch die Verklottung nahm gleichzeitig der Druck am arteriellen Zulaufdrucksensor (9) stark zu, da ein erhöhter Eingangsdruck vorlag. Die Fig.4 zeigt den normalen Druckverlauf während der Therapie, der ohne eine Verklottung weitestgehend stabil ist. Erst durch die Verklottung kommt es zu einem erhöhten Durchflusswiderstand, durch den der Druck am arteriellen Zulaufdrucksensor (9) zunimmt. Der Balken repräsentiert dabei einen voreingestellten Grenzwert von +50 mmHg (66,7 hPa).

Auch dieser Grenzwert wurde mit einem gemessenen Druck von 180mmHg (240 hPa) überschritten. Somit konnte die Verklottung des Dialysators zum Zeitpunkt t = 200min sicher während der laufenden Therapie erkannt werden.

**Patentansprüche**

1.  Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein Toleranzbereich um die Basislinie und ein Toleranzbereich für die gemessenen UV-Absorbanzen berechnet wird und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{(gemessen)}$) innerhalb des berechneten Toleranzbereichs um die Basislinie liegt und ob die gemessene UV-Absorbanz ($A_{y+1(gemessen)}$) innerhalb des berechneten Toleranzbereichs für die berechnete UV-Absorbanz ($A_{y+1(berechnet)}$) liegt, wobei Verklottung detektiert wird, wenn der gemessene Druckmesswert ($P_{(gemessen)}$) außerhalb des berechneten Toleranzbereichs um die Basislinie liegt und die gemessene UV-Absorbanz ($A_{y+1(gemessen)}$) außerhalb des berechneten Toleranzbereichs für die berechnete UV-Absorbanz ($A_{y+1(berechnet)}$) liegt.

2.  Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein oberer Grenzwert über der Basislinie und ein unterer Grenzwert für die gemessenen UV-Absorbanzen berechnet wird und aus einer vordefinierten Anzahl (m) bestimmter UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{(gemessen)}$) den Grenzwert über der Basislinie übersteigt und ob die bestimmte UV-Absorbanz ($A_{y+1(gemessen)}$) den Grenzwert für die berechnete UV-Absorbanz ($A_{y+1(berechnet)}$) unterschreitet, wobei Verklottung detektiert wird, wenn der gemessene Druckmesswert ($P_{(gemessen)}$) den Grenzwert über der Basislinie übersteigt und die gemessene UV-Absorbanz ($A_{y+1(gemessen)}$) den Grenzwert für die berechnete UV-Absorbanz ($A_{y+1(berechnet)}$) unterschreitet.

3.  Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz durch Messung der Transmission der eingestrahlten UV-Strahlung bestimmt wird und aus einer vordefinierten Anzahl (n) gemessener Druckmesswerte ($P_x$), worin x die Anzahl an Druckmessungen bezeichnet, eine als Basislinie bezeichnete Ausgleichsgerade berechnet wird, und ein oberer Grenzwert über der Basislinie und ein oberer Grenzwert für die gemessene Transmission berechnet wird und aus einer vordefinierten Anzahl (m) gemessener Transmissionen ($T_y$) durch Extrapolation eine zukünftige Transmission ($T_{y+1(berechnet)}$) berechnet wird, worin y die Anzahl an UV-Messungen bezeichnet und bestimmt wird, ob der gemessene Druckmesswert ($P_{(gemessen)}$) den Grenzwert über der Basislinie übersteigt und ob die gemessene Transmission ($T_{y+1(gemessen)}$) den Grenzwert für die berechnete Transmission ($T_{y+1(berechnet)}$) übersteigt, wobei Verklottung detektiert wird, wenn der gemessene Druckmesswert ($P_{(gemessen)}$) den Grenzwert über der Basislinie übersteigt und die gemessene Transmission ($T_{y+1(gemessen)}$) den Grenzwert für die berechnete Transmission ($T_{y+1(berechnet)}$) übersteigt.

4.  Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und ein Toleranzbereich für die gemessenen Druckmesswerte und ein Toleranzbereich für die gemessenen UV-Absorbanzen berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{x+1(gemessen)}$) innerhalb des berechneten Toleranzbereichs für den berechneten Druckmesswert ($P_{x+1(berechnet)}$) liegt und ob die gemessene UV-Absorbanz ($A_{y+1(gemessen)}$) innerhalb des berechneten Toleranzbereichs für die berechnete UV-Absorbanz ($A_{y+1(berechnet)}$) liegt, wobei Verklottung detektiert wird, wenn der gemessene Druckmesswert ($P_{x+1(gemessen)}$) außerhalb des berechneten Toleranzbereichs für den berechneten Druckmesswert ($P_{x+1(berechnet)}$) liegt und die gemessene UV-Absorbanz ($A_{y+1(gemessen)}$) außerhalb des berechneten Toleranzbereichs für die berechnete UV-Absorbanz ($A_{y+1(berechnet)}$) liegt.

5. Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz gemessen wird und ein oberer Grenzwert für die gemessenen Druckmesswerte und ein unterer Grenzwert für die gemessenen UV-Absorbanzen berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet wird, worin y die Anzahl an UV-Messungen bezeichnet und bestimmt wird, ob der gemessene Druckmesswert ($P_{x+1(gemessen)}$) den Grenzwert für den berechneten Druckmesswert ($P_{x+1(berechnet)}$) übersteigt und ob die bestimmte UV-Absorbanz ($A_{y+1(gemessen)}$) den Grenzwert für die berechnete UV-Absorbanz ($A_{y+1(berechnet)}$) unterschreitet, wobei Verklottung detektiert wird, wenn der gemessene Druckmesswert ($P_{x+1(gemessen)}$) den Grenzwert für den berechneten Druckmesswert ($P_{x+1(berechnet)}$) übersteigt und die gemessene UV-Absorbanz ($A_{y+1(gemessen)}$) den Grenzwert für die berechnete UV-Absorbanz ($A_{y+1(berechnet)}$) unterschreitet.

6. Verfahren zur Detektion von Verklottung des Dialysators in einer Vorrichtung zur extrakorporalen Reinigung von Blut, wobei blutseitig an mindestens einem Drucksensor der Druck gemessen wird und im Dialysatabfluss die UV-Absorbanz mindestens einer harnpflichtigen Substanz durch Messung der Transmission der eingestrahlten UV-Strahlung gemessen wird und ein oberer Grenzwert für die gemessenen Druckmesswerte und ein oberer Grenzwert für die gemessene Transmission berechnet wird und aus einer vordefinierten Anzahl (n) gemessener Druckmesswerte ($P_x$) durch Extrapolation ein zukünftiger Druckmesswert ($P_{x+1(berechnet)}$) berechnet wird, worin x die Anzahl an Druckmessungen bezeichnet und aus einer vordefinierten Anzahl (m) gemessener Transmissionen ($T_y$) durch Extrapolation eine zukünftige Transmission ($T_{y+1(berechnet)}$) berechnet wird, worin y die Anzahl an UV-Messungen bezeichnet und bestimmt wird, ob der gemessene Druckmesswert ($P_{x+1(gemessen)}$) den Grenzwert für den berechneten Druckmesswert ($P_{x+1(berechnet)}$) übersteigt und ob die gemessene Transmission ($T_{y+1(gemessen)}$) den Grenzwert für die berechnete Transmission ($T_{y+1(berechnet)}$) übersteigt, wobei Verklottung detektiert wird, wenn der gemessene Druckmesswert ($P_{x+1(gemessen)}$) den Grenzwert für den berechneten Druckmesswert ($P_{x+1(berechnet)}$) übersteigt und die gemessene Transmission ($T_{y+1(gemessen)}$) den Grenzwert für die berechnete Transmission ($T_{y+1(berechnet)}$) übersteigt.

7. Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
- einem Zulauf für frische Dialysierflüssigkeit,
- einem Ablauf für verbrauchte Dialysierflüssigkeit,
- einer in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte Toleranzbereiche um die Basislinie für die Druckmesswerte und für die gemessenen Absorbanzen hinterlegt sind,
- eine Datenverarbeitungseinheit, welche aus einer vordefinierten Anzahl (n) gemessener Druckmesswerte ($P_x$), worin x die Anzahl an Druckmessungen bezeichnet, eine als Basislinie bezeichnete Ausgleichsgerade berechnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{(gemessen)}$) innerhalb des festgelegten Toleranzbereichs um die Basislinie liegt und ob die gemessene UV-Absorbanz ($A_{y+1(gemessen)}$) innerhalb des festgelegten Toleranzbereichs für die berechnete UV-Absorbanz ($A_{y+1(berechnet)}$) liegt, wobei Verklottung detektiert und angezeigt wird, wenn der gemessene Druckmesswert ($P_{(gemessen)}$) außerhalb des festgelegten Toleranzbereichs um die Basislinie liegt und die gemessene UV-Absorbanz ($A_{y+1(gemessen)}$) außerhalb des festgelegten Toleranzbereichs für die berechnete UV-Absorbanz ($A_{y+1(berechnet)}$) liegt.

8. Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen über der Basislinie für die Druckmesswerte und festgelegte untere Grenzen für die gemessenen Absorbanzen hinterlegt sind,
- eine Datenverarbeitungseinheit, welche aus einer vordefinierten Anzahl (n) gemessener Druckmesswerte ($P_x$), worin x die Anzahl an Druckmessungen bezeichnet, eine als Basislinie bezeichnete Ausgleichsgerade berechnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{(gemessen)}$) den Grenzwert über der Basislinie übersteigt und ob die gemessene UV-Absorbanz ($A_{y+1(gemessen)}$) den Grenzwert für die berechnete UV-Absorbanz ($A_{y+1(berechnet)}$) unterschreitet, wobei Verklottung detektiert und angezeigt wird, wenn der gemessene Druckmesswert ($P_{(gemessen)}$) den Grenzwert über der Basislinie übersteigt und die gemessene UV-Absorbanz ($A_{y+1(gemessen)}$) den Grenzwert für die berechnete UV-Absorbanz ($A_{y+1(berechnet)}$) unterschreitet.

9. Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabfuhrleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Transmission T der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen über der Basislinie für die Druckmesswerte und für die gemessenen Transmissionen hinterlegt sind,
- eine Datenverarbeitungseinheit, welche aus einer vordefinierten Anzahl (n) gemessener Druckmesswerte ($P_x$), worin x die Anzahl an Druckmessungen bezeichnet, eine als Basislinie bezeichnete Ausgleichsgerade berechnet und aus einer vordefinierten Anzahl (m) gemessener Transmissionen ($T_y$) durch Extrapolation eine zukünftige Transmission ($T_{y+1(berechnet)}$) berechnet, worin y die Anzahl an UV-Messungen bezeichnet und bestimmt, ob der gemessene Druckmesswert ($P_{(gemessen)}$) den Grenzwert über der Basislinie übersteigt und ob die gemessene Transmission ($T_{y+1(gemessen)}$) den Grenzwert für die berechnete Transmission ($T_{y+1(berechnet)}$) übersteigt, wobei Verklottung detektiert und angezeigt wird, wenn der gemessene Druckmesswert ($P_{(gemessen)}$) den Grenzwert über der Basislinie übersteigt und die gemessene Transmission ($T_{y+1(gemessen)}$) den Grenzwert für die berechnete Transmission ($T_{y+1(berechnet)}$) übersteigt.

10. Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
- einen Zulauf für frische Dialysierflüssigkeit,
- einen Ablauf für verbrauchte Dialysierflüssigkeit,

- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte Toleranzbereiche für die gemessenen Druckmesswerte und für die gemessenen Absorbanzen hinterlegt sind,
- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{x+1(gemessen)}$) innerhalb des festgelegten Toleranzbereichs für den berechneten Druckmesswert ($P_{x+1(berechnet)}$) liegt und ob die gemessene UV-Absorbanz ($A_{y+1(gemessen)}$) innerhalb des festgelegten Toleranzbereichs für die berechnete UV-Absorbanz ($A_{y+1(berechnet)}$) liegt, wobei Verklottung detektiert und angezeigt wird, wenn der gemessene Druckmesswert ($P_{x+1(gemessen)}$) außerhalb des festgelegten Toleranzbereichs für den berechneten Druckmesswert ($P_{x+1(berechnet)}$) liegt und die gemessene UV-Absorbanz ($A_{y+1(gemessen)}$) außerhalb des festgelegten Toleranzbereichs für die berechnete UV-Absorbanz ($A_{y+1(berechnet)}$) liegt.

**11.** Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Absorbanz A der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen für die gemessenen Druckmesswerte und festgelegte untere Grenzen für die gemessenen Absorbanzen hinterlegt sind,
- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener UV-Absorbanzen ($A_y$) durch Extrapolation eine zukünftige UV-Absorbanz ($A_{y+1(berechnet)}$) berechnet, worin y die Anzahl an UV-Messungen bezeichnet und bestimmt, ob der gemessene Druckmesswert ($P_{x+1(gemessen)}$) den Grenzwert für den berechneten Druckmesswert ($P_{x+1(berechnet)}$) übersteigt und ob die gemessene UV-Absorbanz ($A_{y+1(gemessen)}$) den Grenzwert für die berechnete UV-Absorbanz ($A_{y+1(berechnet)}$) unterschreitet, wobei Verklottung detektiert und angezeigt wird, wenn der gemessene Druckmesswert ($P_{x+1(gemessen)}$) den Grenzwert für den berechneten Druckmesswert ($P_{x+1(berechnet)}$) übersteigt und die gemessene UV-Absorbanz ($A_{y+1(gemessen)}$) den Grenzwert für die berechnete UV-Absorbanz ($A_{y+1(berechnet)}$) unterschreitet.

**12.** Vorrichtung zur extrakorporalen Reinigung von Blut umfassend:

- einen Dialysator, der durch eine semipermeable Membran in eine erste und zweite Kammer geteilt ist, wobei die erste Kammer in einem Dialysierflüssigkeitsweg angeordnet ist und die zweite Kammer mittels einer Blutzuführleitung und einer Blutabführleitung mit dem Blutkreislauf eines Patienten verbindbar ist,
- einen Zulauf für frische Dialysierflüssigkeit,
- einen Ablauf für verbrauchte Dialysierflüssigkeit,
- eine in dem Ablauf angeordneten UV-Messeinrichtung zur Bestimmung der Transmission T der durch den Ablauf fließenden verbrauchten Dialysierflüssigkeit, wobei die UV-Messeinrichtung wenigstens eine UV-Strahlungsquelle und einen UV-Detektor zur Detektion der Intensität der UV-Strahlung aufweist,
- mindestens einen Drucksensor (PBE und/oder PV) im blutseitigen Kreislauf der Vorrichtung zur extrakorporalen Reinigung von Blut,
- einen Speicher, in welchem festgelegte obere Grenzen für die gemessenen Druckmesswerte und für die gemessenen Transmissionen hinterlegt sind,

- eine Datenverarbeitungseinheit, welche aus einer vordefinierten Anzahl (n) gemessener Druckmesswerte ($P_x$) durch Extrapolation einen zukünftigen Druckmesswert ($P_{x+1(berechnet)}$) berechnet, worin x die Anzahl an Druckmessungen bezeichnet und aus einer vordefinierten Anzahl (m) gemessener Transmissionen ($T_y$) durch Extrapolation eine zukünftige Transmission ($T_{y+1(berechnet)}$) berechnet, worin y die Anzahl an UV-Messungen bezeichnet und bestimmt, ob der gemessene Druckmesswert ($P_{x+1(gemessen)}$) den Grenzwert für den berechneten Druckmesswert ($P_{x+1(berechnet)}$) übersteigt und ob die gemessene Transmission ($T_{y+1(gemessen)}$) den Grenzwert für die berechnete Transmission ($T_{y+1(berechnet)}$) übersteigt, wobei Verklottung detektiert und angezeigt wird, wenn der gemessene Druckmesswert ($P_{x+1(gemessen)}$) den Grenzwert für den berechneten Druckmesswert ($P_{x+1(berechnet)}$) übersteigt und die gemessene Transmission ($T_{y+1(gemessen)}$) den Grenzwert für die berechnete Transmission ($T_{y+1(berechnet)}$) übersteigt.

**Claims**

1. Method of detecting clogging of the dialyzer in an extracorporeal blood cleaning device, whereby the pressure is measured at at least one pressure sensor on the blood side and the UV absorbance of at least one urinary excreted substance in the outflowing dialysate is measured and, from a predefined number (n) of measured pressure measurement values ($P_x$) where x denotes the number of pressure measurements, a best fit line is calculated as a baseline, and a tolerance range around the baseline and a tolerance range for the measured UV absorbance values is calculated and, from a predefined number (m) of measured UV absorbance values ($A_y$) a future UV absorbance value ($A_{y+1(calculated)}$) is calculated by extrapolation, y denoting the number of UV measurements, and it is ascertained whether the measured pressure measurement value ($P_{(measured)}$) lies within the calculated tolerance range around the baseline and whether the measured UV absorbance value ($A_{y+1(measured)}$) lies within the calculated tolerance range for the calculated UV absorbance ($A_{y+1(calculated)}$), and clogging is detected if the measured pressure measurement value ($P_{(measured)}$) lies outside the calculated tolerance range around the baseline and the measured UV absorbance ($A_{y+1(measured)}$) lies outside the calculate tolerance range for the calculated UV absorbance ($A_{y+1(calculated)}$).

2. Method of detecting clogging of the dialyzer in an extracorporeal blood cleaning device, whereby the pressure is measured at at least one pressure sensor on the blood side and the UV absorbance of at least one urinary excreted substance in the outflowing dialysate is measured and, from a predefined number (n) of measured pressure measurement values ($P_x$) where x denotes the number of pressure measurements, a best fit line is calculated as a baseline, and an upper threshold value above the baseline and a lower threshold value for the measured UV absorbance values is calculated and, from a predefined number (m) of measured UV absorbance values ($A_y$) a future UV absorbance ($A_{y+1(calculated)}$) is calculated by extrapolation, y denoting the number of UV measurements, and it is ascertained whether the measured pressure measurement value ($P_{(measured)}$) exceeds the threshold value above the baseline and whether the measured UV absorbance ($A_{y+1(measured)}$) falls below the threshold value for the calculated UV absorbance ($A_{y+1(calculated)}$), and clogging is detected if the measured pressure measurement value ($P_{(measured)}$) exceeds the threshold value above the baseline and the measured UV absorbance ($A_{y+1(measured)}$) falls below the threshold value for the calculated UV absorbance ($A_{y+1(calculated)}$).

3. Method of detecting clogging of the dialyzer in an extracorporeal blood cleaning device, whereby the pressure is measured at at least one pressure sensor on the blood side and the UV absorbance of at least one urinary excreted substance in the outflowing dialysate is determined by measuring the transmission of the irradiated UV radiation and, from a predefined number (n) of measured pressure measurement values ($P_x$), where x denotes the number of pressure measurements, a best fit line is calculated as a baseline, and an upper threshold value above the baseline and an upper threshold value above the baseline and an upper threshold value for the measured transmission is calculated and, from a predefined number (m) of measured transmission values ($T_y$), a future transmission value ($T_{y+1(calculated)}$) is calculated by extrapolation, y being the number of transmission values, and it is ascertained whether the measured pressure measurement value ($P_{(measured)}$) exceeds the threshold value above the baseline and whether the measured transmission ($T_{y+1(measured)}$) exceeds the threshold value for the calculated transmission value ($T_{y+1(calculated)}$), and clogging is detected if the measured pressure measurement value ($P_{(measured)}$) exceeds the threshold value above the baseline and the measured transmission ($T_{y+1(measured)}$) exceeds the threshold value for the calculated transmission ($T_{y+1(calculated)}$).

4. Method of detecting clogging of the dialyzer in an extracorporeal blood cleaning device, whereby the pressure is measured at at least one pressure sensor on the blood side and the UV absorbance of at least one urinary excreted substance in the outflowing dialysate is measured and a tolerance range for the measured pressure measurement values and a tolerance range for the measured UV absorbance values is calculated and, from a predefined number (n) of measured pressure measurement values ($P_x$), a future pressure measurement value ($P_{x+1(calculated)}$) is calculated by extrapolation, x being the number of pressure measurements and, from a predefined number (m) of measured UV absorbance values ($A_y$) a future UV absorbance ($A_{y+1(calculated)}$) is calculated by extrapolation, y denoting the number of UV measurements, and it is ascertained whether the measured pressure measurement value ($P_{x+1(measured)}$) lies within the calculated tolerance range for the calculated pressure measurement value ($P_{x+1(calculated)}$) and whether the measured UV absorbance ($A_{y+1(measured)}$) lies within the calculated tolerance range for the calculated UV absorbance ($A_{y+1(calculated)}$), and clogging is detected if the measured pressure measurement value ($P_{x+1(measured)}$) lies outside the calculated tolerance range for the calculated pressure measurement value ($P_{x+1(calculated)}$) and the measured UV absorbance ($A_{y+1(measured)}$) lies outside the calculate tolerance range for the calculated UV absorbance ($A_{y+1(calculated)}$).

5. Method of detecting clogging of the dialyzer in an extracorporeal blood cleaning device, whereby the pressure is measured at at least one pressure sensor on the blood side and the UV absorbance of at least one urinary excreted substance in the outflowing dialysate is measured and an upper threshold value for the measured pressure measurement values and a lower threshold value for the measured UV absorbance values is calculated and, from a predefined number (n) of measured pressure measurement values ($P_x$) where x denotes the number of pressure measurements, a future pressure measurement value ($P_{x+1(calculated)}$) is calculated by extrapolation and, from a predefined number (m) of measured UV absorbance values ($A_y$), a future UV absorbance ($A_{y+1(calculated)}$) is calculated by extrapolation, y denoting the number of UV measurements, and it is ascertained whether the measured pressure measurement value ($P_{x+1(measured)}$) exceeds the threshold value for the calculated pressure measurement value ($P_{x+1(calculated)}$) and whether the measured UV absorbance ($A_{y+1(measured)}$) falls below the threshold value for the calculated UV absorbance ($A_{y+1(calculated)}$), and clogging is detected if the measured pressure measurement value ($P_{x+1(measured)}$) exceeds the threshold value for the calculated pressure measurement value ($P_{x+1(calculated)}$) and the measured UV absorbance ($A_{y+1(mearured)}$) falls below the threshold value for the calculated UV absorbance ($A_{y+1(calculated)}$).

6. Method of detecting clogging of the dialyzer in an extracorporeal blood cleaning device, whereby the pressure is measured at at least one pressure sensor on the blood side and the UV absorbance of at least one urinary excreted substance in the outflowing dialysate is determined by measuring the transmission of the irradiated UV radiation, and an upper threshold value for the measured pressure measurement values and an upper threshold for the measured transmission is calculated and, from a predefined number (n) of measured pressure measurement values ($P_x$), a future pressure measurement value ($P_{x+1(calculated)}$) is calculated by extrapolation, x being the number of pressure measurements and, from a predefined number (m) of measured transmission values ($T_y$), a future transmission value ($T_{y+1(calculated)}$) is calculated by extrapolation, y being the number of UV measurements, and it is ascertained whether the measured pressure measurement value ($P_{x+1(measured)}$) exceeds the threshold value for the calculated pressure measurement value ($P_{x+1(calculated)}$) and whether the measured transmission ($T_{y+1(measured)}$) exceeds the threshold value for the calculated transmission value ($T_{y+1(calculated)}$), and clogging is detected if the measured pressure measurement value ($P_{x+1(measured)}$) exceeds the threshold value for the calculated pressure measurement value ($P_{x+1(calculated)}$) and the measured transmission ($T_{y+1(measured)}$) exceeds the threshold value for the calculated transmission value ($T_{y+1(calculated)}$).

7. Extracorporeal blood cleaning device comprising:

   - a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,
   - an intake line for fresh dialysis fluid,
   - a discharge for used dialysis fluid,
   - a UV measuring device disposed in the discharge for determining the absorbance A of the used dialysis fluid flowing through the discharge, and the UV measuring device has at least a UV radiation source and a UV detector for detecting the intensity of the UV radiation,
   - at least one pressure sensor (PBE and/or PV) in the blood-side circuit of the extracorporeal blood cleaning device,
   - a memory in which set tolerance ranges around the baseline for the pressure measurement values and for the measured absorbance values are stored,
   - a data processing unit which, from a predefined number (n) of measured pressure measurement values ($P_x$) where x denotes the number of pressure measurements, calculates a best fit line as a baseline and, from a predefined number (m) of measured UV absorbance values ($A_y$), calculates a future UV absorbance value ($A_{y+1(calculated)}$) by extrapolation, y denoting the number of UV measurements, and ascertains whether the measured pressure measurement value ($P_{(measured)}$) lies within the set tolerance range around the baseline and whether the measured UV absorbance value ($A_{y+1(measured)}$) lies within the set tolerance range for the calculated UV absorbance ($A_{y+1(calculated)}$), and clogging is detected and indicated if the measured pressure measurement value ($P_{(measured)}$) lies outside the set tolerance range around the baseline and the measured UV absorbance ($A_{y+1(measured)}$) lies outside the set tolerance range for the calculated UV absorbance ($A_{y+1(calculated)}$).

8. Extracorporeal blood cleaning device comprising:

- a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,
- an intake line for fresh dialysis fluid,
- a discharge for used dialysis fluid,
- a UV measuring device disposed in the discharge for determining the absorbance A of the used dialysis fluid flowing through the discharge, and the UV measuring device has at least a UV radiation source and a UV detector for detecting the intensity of the UV radiation,
- at least one pressure sensor (PBE and/or PV) in the blood-side circuit of the extracorporeal blood cleaning device,
- a memory in which set upper limits above the baseline for the pressure measurement values and set lower limits for the measured absorbance values are stored,
- a data processing unit which, from a predefined number (n) of measured pressure measurement values ($P_x$) where x denotes the number of pressure measurements, calculates a best fit line as a baseline and, from a predefined number (m) of measured UV absorbance values ($A_y$), calculates a future UV absorbance value ($A_{y+1(calculated)}$) by extrapolation, y denoting the number of UV measurements, and ascertains whether the measured pressure measurement value ($P_{(measured)}$) exceeds the threshold value above the baseline and whether the measured UV absorbance value ($A_{y+1(measured)}$) falls below the threshold value for the calculated UV absorbance ($A_{y+1(calculated)}$), and clogging is detected and indicated if the measured pressure measurement value ($P_{(measured)}$) exceeds the threshold value above the baseline and the measured UV absorbance ($A_{y+1(measured)}$) falls below the threshold value for the calculated UV absorbance ($A_{y+1(calculated)}$).

9. Extracorporeal blood cleaning device comprising:

- a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,
- an intake line for fresh dialysis fluid,
- a discharge for used dialysis fluid,
- a UV measuring device disposed in the discharge for determining the transmission T of the used dialysis fluid flowing through the discharge, and the UV measuring device has at least a UV radiation source and a UV detector for detecting the intensity of the UV radiation,
- at least one pressure sensor (PBE and/or PV) in the blood-side circuit of the extracorporeal blood cleaning device,
- a memory in which set upper limits above the baseline for the measured pressure measurement values and for the measured transmission values are stored,
- a data processing unit which, from a predefined number (n) of measured pressure measurement values ($P_x$) where x denotes the number of pressure measurements, calculates a best fit line as a baseline and, from a predefined number (m) of measured transmission values ($T_y$), calculates a future transmission value ($T_{y+1(calculated)}$) by extrapolation, y denoting the number of UV measurements, and ascertains whether the measured pressure measurement value ($P_{(measured)}$) exceeds the threshold value above the baseline and whether the measured transmission value ($T_{y+1(measured)}$) exceeds the threshold value for the calculated transmission ($T_{y+1(calculated)}$), and clogging is detected and indicated if the measured pressure measurement value ($P_{(measured)}$) exceeds the threshold value above the baseline and the measured transmission ($T_{y+1(measured)}$) exceeds the threshold value for the calculated transmission ($T_{y+1(calculated)}$).

10. Extracorporeal blood cleaning device comprising:

- a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,

- an intake line for fresh dialysis fluid,

- a discharge for used dialysis fluid,

- a UV measuring device disposed in the discharge for determining the absorbance A of the used dialysis fluid flowing through the discharge, and the UV measuring device has a at least UV radiation source and a UV detector for detecting the intensity of the UV radiation,

- at least one pressure sensor (PBE and/or PV) in the blood-side circuit of the extracorporeal blood cleaning device,

- a memory in which set tolerance ranges for the measured pressure measurement values and for the measured absorbance values are stored,

- a data processing unit which, from a predefined number (n) of measured pressure measurement values ($P_x$) where x denotes the number of pressure measurements, calculates a future pressure measurement value ($P_{x+1(calculated)}$) and, from a predefined number (m) of measured UV absorbance values ($A_y$), calculates a future absorbance value ($A_{y+1(calculated)}$) by extrapolation, y denoting the number of UV measurements, and ascertains whether the measured pressure measurement value ($P_{x+1(measured)}$) lies within the set tolerance range for the calculated pressure measurement value ($P_{x+1(calculated)}$) and whether the measured UV absorbance ($A_{y+1(measured)}$) lies within the set tolerance range for the calculated UV absorbance ($A_{y+1(calculated)}$), and clogging is detected and indicated if the measured pressure measurement value ($P_{x+1(measured)}$) lies outside the set tolerance range for the calculated pressure measurement value ($P_{x+1(calculated)}$) and the measured UV absorbance ($A_{y+1(measured)}$) lies outside the set tolerance range for the calculated UV absorbance ($A_{y+1(calculated)}$).

**11.** Extracorporeal blood cleaning device comprising:

- a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,

- an intake line for fresh dialysis fluid,

- a discharge for used dialysis fluid,

- a UV measuring device disposed in the discharge for determining the absorbance A of the used dialysis fluid flowing through the discharge, and the UV measuring device has at least a UV radiation source and a UV detector for detecting the intensity of the UV radiation,

- at least one pressure sensor (PBE and/or PV) in the blood-side circuit of the extracorporeal blood cleaning device,

- a memory in which set upper limits for the measured pressure measurement values and set lower limits for the measured absorbance values are stored,

- a data processing unit which, from a predefined number (n) of measured pressure measurement values ($P_x$) where x denotes the number of pressure measurements, calculates a future pressure measurement value ($P_{x+1(calculated)}$) and, from a predefined number (m) of measured UV absorbance values ($A_y$), calculates a future UV absorbance value ($A_{y+1(calculated)}$) by extrapolation, y denoting the number of UV measurements, and ascertains whether the measured pressure measurement value ($P_{x+1(measured)}$) exceeds the threshold value for the calculated pressure measurement value ($P_{x+1(calculated)}$) and whether the measured UV absorbance value ($A_{y+1(measured)}$) falls below the threshold value for the calculated UV absorbance ($A_{y+1(calculated)}$), and clogging is detected and indicated if the measured pressure measurement value ($P_{x+1(measured)}$) exceeds the threshold value for the calculated pressure measurement value ($P_{x+1(calculated)}$) and the measured UV absorbance ($A_{y+1(measured)}$) falls below the threshold value for the calculated UV absorbance ($A_{y+1(calculated)}$).

**12.** Extracorporeal blood cleaning device comprising:

- a dialyzer which is divided by means of a semi-permeable membrane into a first and second compartment, and the first compartment is disposed in a dialysis fluid path and the second compartment can be connected to the blood circulatory system of a patient by means of a blood intake line and a blood discharge line,

- an intake line for fresh dialysis fluid,

- a discharge for used dialysis fluid,

- a UV measuring device disposed in the discharge for determining the transmission T of the used dialysis fluid flowing through the discharge, and the UV measuring device has at least a UV radiation source and a UV detector for detecting the intensity of the UV radiation,

- at least one pressure sensor (PBE and/or PV) in the blood-side circuit of the extracorporeal blood cleaning device,

- a memory in which set upper limits for the measured pressure measurement values and for the measured transmission values are stored,

- a data processing unit which, from a predefined number (n) of measured pressure measurement values ($P_x$) where x denotes the number of pressure measurements, calculates a future pressure measurement value ($P_{x+1(calculated)}$) and, from a predefined number (m) of measured transmission values ($T_y$), calculates a future transmission value ($T_{y+1(calculated)}$) by extrapolation, y denoting the number of UV measurements, and ascertains whether the measured pressure measurement value ($P_{x+1(measured)}$) exceeds the threshold value for the calculated pressure measurement value ($P_{x+1(calculated)}$) and whether the measured transmission value ($T_{y+1(measured)}$) exceeds the threshold value for the calculated transmission ($T_{y+1(calculated)}$), and clogging is detected and indicated if the measured pressure measurement value ($P_{x+1(measured)}$) exceeds the threshold value for the calculated pressure measurement value ($P_{x+1(calculated)}$) and the measured transmission ($T_{y+1(measured)}$) exceeds the threshold value for the calculated transmission ($T_{y+1(calculated)}$).

## Revendications

1. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique dans l'évacuation du dialysat est mesurée et une droite de régression, désignée comme ligne de base, est calculée à partir d'un nombre (n) prédéfini de valeurs de mesure de pression ($P_x$) mesurées, x désignant le nombre de mesures de la pression, et une zone de tolérances autour de la ligne de base et une zone de tolérances pour les absorbances UV mesurées sont calculées, et une absorbance UV ($A_{y+1(berechnet}[1]{)}$) future est calculée par extrapolation à partir d'un nombre (m) prédéfini d'absorbances UV ($A_y$) mesurées, y désignant le nombre de mesures UV, et il est déterminé si la valeur de mesure de pression mesurée ($P_{(gemessen}[2]{)}$) se situe à l'intérieur de la zone de tolérances calculée autour de la ligne de base et si l'absorbance UV mesurée ($A_{y+1(gemessen)}$) se situe à l'intérieur de la zone de tolérances calculée pour l'absorbance UV calculée ($A_{y+1(berechnet)}$), un engorgement étant détecté lorsque la valeur de mesure de pression mesurée ($P_{(gemessen)}$) se situe à l'extérieur de la zone de tolérances calculée autour de la ligne de base et l'absorbance UV mesurée ($A_{y+1(gemessen)}$) se situe à l'extérieur de la zone de tolérances calculée pour l'absorbance UV calculée ($A_{y+1(berechnet)}$).

2. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique est mesurée dans l'évacuation du dialysat, et une droite de régression, désignée comme ligne de base, est calculée à partir d'un nombre (n) prédéfini de valeurs de mesure de pression ($P_x$) mesurées, x désignant le nombre de mesures de la pression, et une valeur limite supérieure au-dessus de la ligne de base et une valeur limite inférieure pour les absorbances UV mesurées sont calculées, et une absorbance UV ($A_{y+1(berechnet)}$) future est calculée par extrapolation à partir d'un nombre (m) prédéfini d'absorbances UV ($A_y$) déterminées, y désignant le nombre de mesures UV, et il est déterminé si la valeur de mesure de pression mesurée ($P_{(gemessen)}$) est supérieure à la valeur limite au-dessus de la ligne de base et si

[1] Berechnet = calculée

[2] Gemessen = mesurée l'absorbance UV déterminée ($A_{y+1(gemessen)}$) est inférieure à la valeur limite pour l'absorbance UV calculée ($A_{y+1(berechnet)}$), un engorgement étant détecté lorsque la valeur de mesure de pression mesurée ($P_{(gemessen)}$) est supérieure à la valeur limite au-dessus de la ligne de base et l'absorbance UV mesurée ($A_{y+1(gemessen)}$) est inférieure à la valeur limite pour l'absorbance UV calculée ($A_{y+1(berechnet)}$).

3. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique est déterminée dans l'évacuation du dialysat par la mesure de la transmission du rayonnement UV injecté, et une droite de régression, désignée comme ligne de base, est calculée à partir d'un nombre (n) prédéfini de valeurs de mesure de pression ($P_x$) mesurées, x désignant le nombre de mesures de la pression, et une valeur limite supérieure au-dessus de la ligne de base et une valeur limite supérieure pour la transmission mesurée sont calculées, et une transmission ($T_{y+1(berechnet)}$) future est calculée par extrapolation à partir d'un nombre (m) prédéfini de transmissions ($T_y$) mesurées, y désignant le nombre de mesures UV, et il est déterminé si la valeur de mesure de pression mesurée ($P_{(gemessen)}$) est supérieure à la valeur limite au-dessus de la ligne de base et si la transmission mesurée ($T_{y+1(gemessen)}$) est supérieure à la valeur limite pour la transmission calculée ($T_{y+1(berechnet)}$), un engorgement étant détecté lorsque la valeur de mesure de pression mesurée ($P_{(gemessen)}$) est supérieure à la valeur limite au-dessus de la ligne de base et la transmission mesurée ($T_{y+1(gemessen)}$) est supérieure à la valeur limite pour la transmission calculée ($T_{y+1(berechnet)}$).

4. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique est mesurée dans l'évacuation du dialysat, et une zone de tolérances pour les valeurs de mesure de pression mesurées et une zone de tolérances pour les absorbances UV mesurées sont calculées, et une valeur de mesure de pression future ($P_{x+1(berechnet)}$) est calculée par extrapolation à partir d'un nombre (n) prédéfini de valeurs de mesure ($P_x$) mesurées, x désignant le nombre de mesures de la pression, et une absorbance UV ($A_{y+1(berechnet)}$) future est calculée par extrapolation à partir d'un nombre (m) prédéfini d'absorbances UV ($A_y$) mesurées, y désignant le nombre de mesures UV, et il est déterminé si la valeur de mesure de pression mesurée ($P_{x+1(gemessen)}$) se situe à l'intérieur de la zone de tolérances calculée pour la valeur de mesure de pression calculée ($P_{x+1(berechnet)}$) et si l'absorbance UV mesurée ($A_{y+1(gemessen)}$) se situe à l'intérieur de la zone de tolérances calculée pour l'absorbance UV calculée ($A_{y+1(berechnet)}$). un engorgement étant détecté lorsque la valeur de mesure de pression mesurée ($P_{x+1(gemessen)}$) se situe à l'extérieur de la zone de tolérances calculée pour la valeur de mesure de pression calculée ($P_{x+1(berechnet)}$) et l'absorbance UV mesurée ($A_{y+1(gemessen)}$) se situe à l'extérieur de la zone de tolérances calculée pour l'absorbance UV calculée ($A_{y+1(berechnet)}$).

5. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique est mesurée dans l'évacuation du dialysat, et une valeur limite supérieure pour les valeurs de mesure de pression mesurées et une valeur limite inférieure pour les absorbances UV mesurées sont calculées, et une valeur de mesure de pression future ($P_{x+1(berechnet)}$) est calculée par extrapolation à partir d'un nombre (n) prédéfini de valeurs de mesure ($P_x$) mesurées, x désignant le nombre de mesures de la pression, et une absorbance UV ($A_{y+1(berechnet)}$) future est calculée par extrapolation à partir d'un nombre (m) prédéfini d'absorbances UV ($A_y$) mesurées, y désignant le nombre de mesures UV, et il est déterminé si la valeur de mesure de pression mesurée ($P_{x+1(gemessen)}$) est supérieure à la valeur limite pour la valeur de mesure de pression calculée ($P_{x+1(berechnet)}$) et si l'absorbance UV déterminée ($A_{y+1(gemessen)}$) est inférieure à la valeur limite pour l'absorbance UV calculée ($A_{y+1(berechnet)}$), un engorgement étant détecté lorsque la valeur de mesure de pression mesurée ($P_{x+1(gemessen)}$) est supérieure à la valeur limite pour la valeur de mesure de pression calculée ($P_{x+1(berechnet)}$) et l'absorbance UV mesurée ($A_{y+1(gemessen)}$) est inférieure à la valeur limite pour l'absorbance UV calculée ($A_{y+1(berechnet)}$).

6. Procédé pour détecter un engorgement du dialyseur dans un dispositif d'épuration extra-corporelle du sang, dans lequel la pression est mesurée du côté du sang sur au moins un capteur de pression et l'absorbance UV d'au moins une toxine urémique est mesurée dans l'évacuation du dialysat à l'appui de la mesure de la transmission du rayonnement UV injecté, et une valeur limite supérieure pour les valeurs de mesure de pression mesurées et une valeur limite supérieure pour la transmission mesurée sont calculées, et une valeur de mesure de pression future ($P_{x+1(berechnet)}$) est calculée par extrapolation à partir d'un nombre (n) prédéfini de valeurs de mesure ($P_x$) mesurées, x désignant le nombre de mesures de la pression, et une transmission ($T_{y+1(berechnet)}$) future est calculée par extrapolation à partir d'un nombre (m) prédéfini de transmissions ($T_y$) mesurées, y désignant le nombre de mesures UV, et il est déterminé si la valeur de mesure de pression mesurée ($P_{x+1(gemessen)}$) est supérieure à la valeur limite pour la valeur de mesure de pression calculée ($P_{x+1(berechnet)}$) et si la transmission mesurée ($T_{y+1(gemessen)}$) est supérieure à la valeur limite pour la transmission calculée ($T_{y+1(berechnet)}$), un engorgement étant détecté lorsque la valeur de mesure de pression mesurée ($P_{x+1(gemessen)}$) est supérieure à la valeur limite pour la valeur de mesure de pression calculée ($P_{x+1(berechnet)}$) et la transmission mesurée ($T_{y+1(gemessen)}$) est supérieure à la valeur limite pour la transmission calculée ($T_{y+1(berechnet)}$).

7. Dispositif pour l'épuration extra-corporelle du sang, comportant :

   - un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
   - une admission pour le liquide de dialyse non altéré,
   - une évacuation pour le liquide de dialyse vicié,
   - un dispositif de mesure UV, disposé dans l'évacuation et destiné à déterminer l'absorbance A du liquide de dialyse vicié circulant à travers l'évacuation, le dispositif de mesure UV comportant au moins une source de

28

rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des zones de tolérance définies autour de la ligne de base pour les valeurs de mesure de pression et pour les absorbances mesurées,
- une unité de traitement des données, qui calcule une droite de régression, désignée comme ligne de base, à partir d'un nombre (n) prédéfini de valeurs de mesure de pression mesurées ($P_x$), x désignant le nombre de mesures de la pression, et calcule une absorbance UV ($A_{y+1(berechnet)}$) future par extrapolation à partir d'un nombre (m) prédéfini d'absorbances UV ($A_y$) mesurées, y désignant le nombre de mesures UV, et détermine si la valeur de mesure de pression mesurée ($P_{(gemessen)}$) se situe à l'intérieur de la zone de tolérances définie autour de la ligne de base et si l'absorbance UV mesurée ($A_{y+1(gemessen)}$) se situe à l'intérieur de la zone de tolérances définie pour l'absorbance UV calculée ($A_{y+1(berechnet)}$), un engorgement étant détecté et signalé lorsque la valeur de mesure de pression mesurée ($P_{(gemessen)}$) se situe à l'extérieur de la zone de tolérances définie autour de la ligne de base et l'absorbance UV mesurée ($A_{y+1(gemessen)}$) se situe à l'extérieur de la zone de tolérances définie pour l'absorbance UV calculée ($A_{y+1(berechnet)}$).

8. Dispositif pour l'épuration extra-corporelle du sang, comportant :

- un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
- une admission pour le liquide de dialyse non altéré,
- une évacuation pour le liquide de dialyse vicié,
- un dispositif de mesure UV, disposé dans l'évacuation et destiné à déterminer l'absorbance A du liquide de dialyse vicié circulant à travers l'évacuation, le dispositif de mesure UV comportant au moins une source de rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des limites supérieures définies au-dessus de la ligne de base pour les valeurs de mesure de pression et des limites inférieures définies pour les absorbances mesurées,
- une unité de traitement des données, qui calcule une droite de régression, désignée comme ligne de base, à partir d'un nombre (n) prédéfini de valeurs de mesure de pression mesurées ($P_x$), x désignant le nombre de mesures de la pression, et calcule une absorbance UV ($A_{y+1(berechnet)}$) future par extrapolation à partir d'un nombre (m) prédéfini d'absorbances UV ($A_y$) mesurées, y désignant le nombre de mesures UV, et détermine si la valeur de mesure de pression mesurée ($P_{(gemessen)}$) est supérieure à la valeur limite au-dessus de la ligne de base et si l'absorbance UV mesurée ($A_{y+1(gemessen)}$) est inférieure à la valeur limite pour l'absorbance UV calculée ($A_{y+1(berechnet)}$), un engorgement étant détecté et signalé lorsque la valeur de mesure de pression mesurée ($P_{(gemessen)}$) est supérieure à la valeur limite au-dessus de la ligne de base et l'absorbance UV mesurée ($A_{y+1(gemessen)}$) est inférieure à la valeur limite pour l'absorbance UV calculée ($A_{y+1(berechnet)}$).

9. Dispositif pour l'épuration extra-corporelle du sang, comportant :

- un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
- une admission pour le liquide de dialyse non altéré,
- une évacuation pour le liquide de dialyse vicié,
- un dispositif de mesure UV, disposé dans l'évacuation et destiné à déterminer la transmission T du liquide de dialyse vicié circulant à travers l'évacuation, le dispositif de mesure UV comportant au moins une source de rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des limites supérieures définies au-dessus de la ligne de base pour les valeurs de mesure de pression et pour les transmissions mesurées,
- une unité de traitement des données, qui calcule une droite de régression, désignée comme ligne de base, à partir d'un nombre (n) prédéfini de valeurs de mesure de pression mesurées ($P_x$), x désignant le nombre de mesures de la pression, et calcule une transmission ($T_{y+1(berechnet)}$) future par extrapolation à partir d'un nombre (m) prédéfini de transmissions ($T_y$) mesurées, y désignant le nombre de mesures UV, et détermine si la valeur de mesure de pression mesurée ($P_{(gemessen)}$) est supérieure à la valeur limite au-dessus de la ligne de base et si la transmission mesurée ($T_{y+1(gemessen)}$) est supérieure à la valeur limite pour la transmission calculée ($T_{y+1(berechnet)}$), un engorgement étant détecté et signalé lorsque la valeur de mesure de pression mesurée ($P_{(gemessen)}$) est supérieure à la valeur limite au-dessus de la ligne de base et la transmission mesurée ($T_{y+1(gemessen)}$) est supérieure à la valeur limite pour la transmission calculée ($_{Ty+1(berechnet)}$).

**10.** Dispositif pour l'épuration extra-corporelle du sang, comportant :

- un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
- une admission pour le liquide de dialyse non altéré,
- une évacuation pour le liquide de dialyse vicié,
- un dispositif de mesure UV, disposé dans l'évacuation et destiné à déterminer l'absorbance A du liquide de dialyse vicié circulant à travers l'évacuation, le dispositif de mesure UV comportant au moins une source de rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des zones de tolérances définies pour les valeurs de mesure de pression mesurées et pour les absorbances mesurées,
- une unité de traitement des données, qui calcule une valeur de mesure de pression future ($P_{x+1(berechnet)}$) par extrapolation à partir d'un nombre (n) prédéfini de valeurs de mesure de pression ($P_x$) mesurées, x désignant le nombre de mesures de la pression, et calcule une absorbance UV ($A_{y+1(berechnet)}$) future par extrapolation à partir d'un nombre (m) prédéfini d'absorbances UV ($A_y$) mesurées, y désignant le nombre de mesures UV, et détermine si la valeur de mesure de pression mesurée ($P_{x+1(gemessen)}$) se situe à l'intérieur de la zone de tolérances définie pour la valeur de mesure de pression calculée ($P_{x+1(berechnet)}$) et si l'absorbance UV mesurée ($A_{y+1(gemessen)}$) se situe à l'intérieur de la zone de tolérances définie pour l'absorbance UV calculée ($A_{y+1(berechnet)}$), un engorgement étant détecté et signalé lorsque la valeur de mesure de pression mesurée ($P_{x+1(gemessen)}$) se situe à l'extérieur de la zone de tolérances définie pour la valeur de mesure de pression calculée ($P_{x+1(berechnet)}$) et l'absorbance UV mesurée ($A_{y+1(gemessen)}$) se situe à l'extérieur de la zone de tolérances définie pour l'absorbance UV calculée ($A_{y+1(berechnet)}$).

**11.** Dispositif pour l'épuration extra-corporelle du sang, comportant :

- un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
- une admission pour le liquide de dialyse non altéré,
- une évacuation pour le liquide de dialyse vicié,
- un dispositif de mesure UV, disposé dans l'évacuation et destiné à déterminer l'absorbance A du liquide de dialyse vicié circulant à travers l'évacuation, le dispositif de mesure UV comportant au moins une source de rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des limites supérieures définies pour les valeurs de mesure de pression mesurées et des limites inférieures définies pour les absorbances mesurées,
- une unité de traitement des données, qui calcule une valeur de mesure de pression future ($P_{x+1(berechnet)}$) par extrapolation à partir d'un nombre (n) prédéfini de valeurs de mesure de pression ($P_x$) mesurées, x désignant le nombre de mesures de la pression, et calcule une absorbance UV ($A_{y+1(berechnet)}$) future par extrapolation à partir d'un nombre (m) prédéfini d'absorbances UV ($A_y$) mesurées, y désignant le nombre de mesures UV, et détermine si la valeur de mesure de pression mesurée ($P_{x+1(gemessen)}$) est supérieure à la valeur limite pour la valeur de mesure de pression calculée ($P_{x+1(berechnet)}$) et si l'absorbance UV mesurée ($A_{y+1(gemessen)}$) est inférieure à la valeur limite pour l'absorbance UV calculée ($A_{y+1(berechnet)}$), un engorgement étant détecté et signalé lorsque la valeur de mesure de pression mesurée ($P_{x+1(gemessen)}$) est supérieure à la valeur limite pour la valeur de mesure de pression calculée ($P_{x+1(berechnet)}$) et l'absorbance UV mesurée ($A_{y+1(gemessen)}$) est inférieure à la valeur limite pour l'absorbance UV calculée ($A_{y+1(berechnet)}$).

12. Dispositif pour l'épuration extra-corporelle du sang, comportant :

- un dialyseur qui est divisé en une première et une deuxième chambre par une membrane semi-perméable, la première chambre étant disposée dans une voie de liquide de dialyse et la deuxième chambre pouvant être reliée à la circulation sanguine d'un patient par une ligne d'acheminement du sang et une ligne d'évacuation du sang,
- une admission pour le liquide de dialyse non altéré,
- une évacuation pour le liquide de dialyse vicié,
- un dispositif de mesure UV, disposé dans l'évacuation et destiné à déterminer la transmission T du liquide de dialyse vicié circulant à travers l'évacuation, le dispositif de mesure UV comportant au moins une source de rayonnement UV et un détecteur UV permettant de détecter l'intensité du rayonnement UV,
- au moins un capteur de pression (PBE et/ou PV) dans le circuit du côté sang du dispositif d'épuration extra-corporelle du sang,
- une mémoire, dans laquelle sont stockées des limites supérieures pour les valeurs de mesure de pression mesurées et pour les transmissions mesurées,
- une unité de traitement des données, qui calcule une valeur de mesure de pression future ($P_{x+1(berechnet)}$) par extrapolation à partir d'un nombre (n) prédéfini de valeurs de mesure ($P_x$) mesurées, x désignant le nombre de mesures de la pression, et calcule une transmission ($T_{y+1(berechnet)}$) future par extrapolation à partir d'un nombre (m) prédéfini de transmissions ($T_y$) mesurées, y désignant le nombre de mesures UV, et détermine si la valeur de mesure de pression mesurée ($P_{x+1(gemessen)}$) est supérieure à la valeur limite pour la valeur de mesure de pression calculée ($P_{x+1(berechnet)}$) et si la transmission mesurée ($T_{y+1(gemessen)}$) est supérieure à la valeur limite pour la transmission calculée ($T_{y+1(berechnet)}$), un engorgement étant détecté et signalé lorsque la valeur de mesure de pression mesurée ($P_{x+1(gemessen)}$) est supérieure à la valeur limite pour la valeur de mesure de pression calculée ($P_{x+1(berechnet)}$) et la transmission mesurée ($T_{y+1(gemessen)}$) est supérieure à la valeur limite pour la transmission calculée ($T_{y+1(berechnet)}$).

**Figur 1**

**Figur 2**

## Figur 3

## Figur 4

**Figur 5**

$y = 43.623x + 588.81$

# EP 2 735 323 B1

**IN DER BESCHREIBUNG AUFGEFÜHRTE DOKUMENTE**

**In der Beschreibung aufgeführte Patentdokumente**

*   WO 2004002553 A1